# EUROPEAN PATENT APPLICATION

(11) **EP 2 280 009 A1**
(43) Date of publication of application: **02.02.2011**
(21) Application number: 09754444.9
(22) Date of filing: 27.05.2009
(51) Int. Cl.: C07D 405/04, A01N 43/40, A01N 43/86, A01P 3/00

(54) **NITROGENOUS HETEROCYCLIC COMPOUNDS AND FUNGICIDES FOR AGRICULTURAL AND HORTICULTURAL USE**

(30) Priority: 28.05.2008 JP 2008140183
(71) Applicant: Nippon Soda Co., Ltd., Tokyo 100-8165 (JP)
(72) Inventor: MITANI, Akira, Odawara-shi Kanagawa 250-0280 (JP); INAGAKI, Jun, Odawara-shi Kanagawa 250-0280 (JP); KUWAHARA, Raito, Odawara-shi Kanagawa 250-0280 (JP); YOKOYAMA, Masahiro, Odawara-shi Kanagawa 250-0280 (JP)
(74) Representative: Wills, Andrew Jonathan
(86) International application number: PCT/JP2009/002346
(87) International publication number: WO 2009/144935

(57) **Abstract**

The present invention provides a novel nitrogen-containing heterocyclic compound and salt thereof, and a fungicide for agricultural and horticultural use that contains at least one thereof as an active ingredient, demonstrates reliable effects and can be used safely. The nitrogen-containing heterocyclic compound of the present invention is represented by the following formula (I) (wherein, R¹ to R⁷ each independently represent, for example, a hydrogen atom, halogen atom or C1-20 alkyl group, X represents a halogen atom, nitro group, unsubstituted or substituted amino group, hydroxyl group or organic group, n represents an integer of 0 to 4, and Z represents, for example, an oxygen atom, sulfur atom or group represented by the formula NR¹⁰ (wherein, R¹⁰ represents, for example, an alkyl group)).

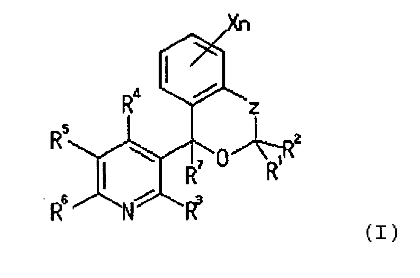

## Description

### TECHNICAL FIELD

The present invention relates to a novel nitrogen-containing heterocyclic compound and salt thereof, and a fungicide for agricultural and horticultural use that contains at least one of these compounds as an active ingredient thereof.
The present application claims priority on Japanese Patent Application No. 2008-140183, filed in Japan on May 28, 2008, the content of which is incorporated herein by reference.

### BACKGROUND ART

In the cultivation of agricultural and horticultural crops, although numerous control agents are used against crop diseases, since their control effects are inadequate, their use has been limited due to the appearance of drug-resistant pathogens, the control agents cause chemical damage or contamination of plants, or from the viewpoint of toxicity to humans, livestock and fish or effects on the environment, few of these control agents can actually be said to be satisfactory. Thus, there is a strong need for the development of a drug that is almost free of these disadvantages and can be used safely.

In relation to the present invention, the following Patent Documents 1 and 2 disclose a quinoline derivative, which has a chemical structure that resembles that of a compound of the present invention, and a fungicide for agricultural and horticultural use that contains the quinoline derivative as an active ingredient thereof. However, there is no description of a compound of the present invention.

### [Prior Art Documents]

### [Patent Documents]

[Patent Document 1] International Publication No. WO 2005/070917
[Patent Document 2] International Publication No. WO 2007/011022

### SUMMARY OF THE INVENTION

### [Problems to be Solved by the Invention]

An object of the present invention is to provide a novel nitrogen-containing heterocyclic compound or salt thereof, and a fungicide for agricultural and horticultural use that contains at least one of these compounds as an active ingredient thereof, demonstrates reliable effects and can be used safely.

### [Means for Solving the Problems]

In order to solve the aforementioned problems, the present invention provides a nitrogen-containing heterocyclic compound represented by the following formula (I), or a salt thereof.

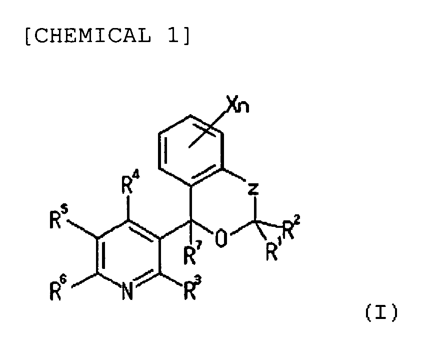

wherein, R¹ and R² each independently represent a hydrogen atom, halogen atom, C1-20 alkyl group, C1-20 haloalkyl group, C2-20 alkenyl group, C2-20 haloalkenyl group, C2-20 alkynyl group, C2-20 haloalkynyl group, C1-20 alkoxy group, C1-20 haloalkoxy group, C3-20 cycloalkyl group, C4-20 cycloalkenyl group, C8-20 cycloalkynyl group, C1-20 acyl group or C1-20 alkoxycarbonyl group, and R¹ and R² may bond together to form an unsubstituted or substituted 5- to 8-membered ring;
R³ to R⁶ each independently represent a hydrogen atom, halogen atom, C1-20 alkyl group, C1-20 haloalkyl group, C2-20 alkenyl group, C2-20 haloalkenyl group, C2-20 alkynyl group, C2-20 haloalkynyl group, C1-20 alkoxy group, C1-20 haloalkoxy group, C3-20 cycloalkyl group, C3-20 cycloalkoxy group, C2-20 alkenyloxy group, C2-20 alkynyloxy group, C1-20alkylthio group, cyano group, nitro group, unsubstituted or substituted amino group, unsubstituted or substituted aryl group, unsubstituted or substituted aralkyl group, unsubstituted or substituted aryloxy group or unsubstituted or substituted heterocyclic group, and R⁴ and R⁵, and R⁵ and R⁶ may bond together to form an unsubstituted or substituted 5- to 8-membered ring;
R⁷ represents a hydrogen atom, halogen atom, hydroxyl group, C1-20 alkyl group, C1-20 haloalkyl group, C2-20 alkenyl group, C2-20 haloalkenyl group, C2-20 alkynyl group, C2-20 haloalkynyl group, C1-20 alkoxy group, C1-20 haloalkoxy group, C3-20 cycloalkyl group, C3-20 cycloalkoxy group, C2-20 alkenyloxy group, C2-20 alkynyloxy group, C1-20 alkylthio group or cyano group;
X represents a halogen atom, nitro group, unsubstituted or substituted amino group, hydroxyl group or organic group;
n represents an integer of 0 to 4;
Z represents an oxygen atom, sulfur atom, group represented by the formula: CR⁸R⁹ or the formula: NR¹⁰;
R⁸and R⁹ each independently represent a hydrogen atom, halogen atom, C1-20 alkyl group, C1-20 haloalkyl group, C2-20 alkenyl group, C2-20 haloalkenyl group, C2-20 alkynyl group, C2-20 haloalkynyl group, C1-20 alkoxy group, C1-20 haloalkoxy group, C3-20 cycloalkyl group, C4-20 cycloalkenyl group, C8-20 cycloalkynyl group, C1-20 acyl group or C1-20 alkoxycarbonyl group, and R⁸ and R⁹ may bond together to form an unsubstituted or substituted 5- to 8-membered ring; and,
R¹⁰ represents a hydrogen atom, C1-20 alkyl group, C2-20 alkenyl group, C2-20 alkynyl group, C1-20 acyl group, C1-20 alkoxycarbonyl group, C1-20 alkylsulfonyl group, C1-20 alkoxy C1-20 alkyl group, C2-20 acyloxy C1-20 alkyl group, unsubstituted or substituted aminocarbonyl group, unsubstituted or substituted arylsulfonyl group or unsubstituted or substituted aminosulfonyl group.

In the nitrogen-containing heterocyclic compound or salt thereof of the present invention, R⁵ and R⁶ preferably bond together to form an aromatic ring.
In addition, the present invention provides a fungicide for agricultural and horticultural use that contains at least one of the nitrogen-containing heterocyclic compound or salt thereof of the present invention as an active ingredient thereof.

### [Effects of the Invention]

The nitrogen-containing heterocyclic compound or salt thereof of the present invention is a novel compound, and is useful as an active ingredient of a fungicide for agricultural and horticultural use that demonstrates reliable effects and can be used safely.
The fungicide for agricultural and horticultural use of the present invention is an agent that has superior control effects, does not cause chemical damage to plants, and has little toxicity on humans, livestock or fish and little effect on the environment.

### BEST MODE FOR CARRYING OUT THE INVENTION

The following provides a detailed explanation of the present invention by dividing into sections of: 1) nitrogen-containing heterocyclic compound represented by formula (I) or salt thereof, and 2) fungicide for agricultural and horticultural use.

### 1) Nitrogen-Containing Heterocyclic Compound Represented by Formula (I) or Salt Thereof

A compound of the present invention is a nitrogen-containing heterocyclic compound represented by the aforementioned formula (I), or a salt thereof. Hydrates, various types of solvates, crystalline polymorphs, R forms, S forms, racemic forms and the like are included in the nitrogen-containing heterocyclic compound of the present invention or salt thereof.
In formula (I), R¹ and R² each independently represent a hydrogen atom, halogen atom, C1-20 alkyl group, C1-20 haloalkyl group, C2-20 alkenyl group, C2-20 haloalkenyl group, C2-20 alkynyl group, C2-20 haloalkynyl group, C1-20 alkoxy group, C1-20 haloalkoxy group, C3-20 cycloalkyl group, C4-20 cycloalkenyl group, C8-20 cycloalkynyl group, C1-20 acyl group or C1-20 alkoxycarbonyl group. R¹ and R² may bond together to form an unsubstituted or substituted 5- to 8-membered ring.

Examples of the "halogen atom" of R¹ and R² include a fluorine atom, chlorine atom, bromine atom and iodine atom.
The "C1-20 alkyl group" of R¹ and R² refers to a linear or branched alkyl group having 1 to 20 carbon atoms. Examples include a methyl group, ethyl group, n-propyl group, i-propyl group, n-butyl group, i-butyl group, s-butyl group, t-butyl group, n-pentyl group, isopentyl group, neopentyl group, 2-methylbutyl group, 2,2-dimethylpropyl group, n-hexyl group, isohexyl group, n-heptyl group, n-octyl group, nonyl group, isononyl group, n-decyl group, 2-ethyloctyl group, 3-ethyloctyl group, 2,3-dimethyloctyl group, 4-propyloctyl group, dodecyl group, 4-ethyldecyl group, 6-ethyldodecyl group, 3,5-dimethyldecyl group, 2,5-dimethyldecyl group, 6-propylnonyl group, 4-butyloctyl group, tridecyl group, tetradecyl group, 2,4,6,8-tetramethyldecyl group, 2,2-diethyldecyl group, 2,5-diethyldecyl group, 4-butyldecyl group, pentadecyl group, hexadecyl group, heptadecyl group, octadecyl group, 2,4,6,8-tetraethyldecyl group, nonadecyl group and icosyl group. Among these, C1-6 alkyl groups are preferable.

The "C1-20 haloalkyl group" of R¹ and R² refers to a group in which a hydrogen atom of a C1-20 alkyl group is substituted with a halogen atom. Examples include a fluoromethyl group, chloromethyl group, bromomethyl group, difluoromethyl group, dichloromethyl group, dibromomethyl group, trifluoromethyl group, trichloromethyl group, tribromomethyl group, 2,2,2-trifluoroethyl group, 2,2,2-trichloroethyl group, pentafluoroethyl group, 4-fluorobutyl group, 4-chlorobutyl group, 3,3,3-trifluoropropyl group, 2,2,2-trifluoro-1-trifluoromethylethyl group, perfluorohexyl group, perchlorohexyl group, perfluorooctyl group, perchlorooctyl group, 2,4,6-trichlorohexyl group, perfluorodecyl group, 2,2,4,4,6,6-hexachlorooctyl group and 2,2,4,4,6,6-hexachloro-3-propyl-octyl group. Among these, C1-6 haloalkyl groups are preferable.

The "C2-20 alkenyl group" of R¹ and R² refers to a linear or branched alkenyl group having 2 to 20 carbon atoms that has a carbon-carbon double bond at one or more locations of an alkyl group. Examples include a vinyl group, 1-propenyl group, allyl group, 1-butenyl group, 2-butenyl group, 3-butenyl group, 1-methyl-2-propenyl group, 2-methyl-2-propenyl group, 1-pentenyl group, 2-pentenyl group, 3-pentenyl group, 4-pentenyl group, 1-methyl-2-butenyl group, 2-methyl-2-butenyl group, 1-hexenyl group, 2-hexenyl group, 3-hexenyl group, 4-hexenyl group, 5-hexenyl group, 1-heptenyl group, 6-heptenyl group, 1-octenyl group, 7-octenyl group, 1-decenyl group, 9-decenyl group, 1-dodecenyl group, 4-dodecenyl group, 1-octadecenyl group and 2-ethyl-1-octadecenyl group. Among these, C2-6 alkenyl groups are preferable.

The "C2-20 haloalkenyl group" of R¹ and R² refers to a linear or branched alkenyl group having 2 to 20 carbon atoms having a carbon-carbon double bond at one or more locations of a haloalkyl group. Examples include a 3-chloro-2-propenyl group, 4-chloro-2-butenyl group, 4,4-dichloro-3-butenyl group, 4,4-difluoro-3-butenyl group, 3,3-dichloro-2-propenyl group, 2,3-dichloro-2-propenyl group, 3,3-difluoro-2-propenyl group, 2,4,6,-trichloro-2-hexenyl group and 3-ethyl-2,4,6-trifluoro-2-hexenyl group. Among these, C2-6 haloalkenyl groups are preferable.

The "C2-20 alkynyl group" of R¹ and R² refers to a linear or branched alkynyl group having 2 to 20 carbon atoms having a carbon-carbon triple bond at one or more locations of an alkyl group. Examples include an ethynyl group, 1-propynyl group, propargyl group, 1-butynyl group, 2-butynyl group, 3-butynyl group, 1-methyl-2-propynyl group, 2-methyl-3-butynyl group, 1-pentynyl group, 2-pentynyl group, 3-pentynyl group, 4-pentynyl group, 1-methyl-2-butynyl group, 2-methyl-3-pentynyl group, 1-hexynyl group, 1,1-dimethyl-2-butynyl group, dodecynyl group, butadecynyl group, heptadecynyl group and 4-ethylhexadecynyl group. Among these, C2-6 alkynyl groups are preferable.

The "C2-20 haloalkynyl group" of R¹ and R² refers to a linear or branched alkynyl group having 2 to 20 carbon atoms having a carbon-carbon double bond at one or more locations of a haloalkyl group. Examples include a 3-chloro-1-propynyl group, 3-chloro-1-butynyl group, 3-bromo-1-butynyl group, 3-bromo-2-propynyl group, 3-iodo-2-propynyl group, 5,5-dichloro-2-methyl-3-pentynyl group, 3-bromo-1-hexynyl group, 4-chloro-1,1-dimethyl-2-butynyl group and 4,4,6,6-tetrafluoro-1-dodecynyl group. Among these, C2-6 haloalkynyl groups are preferable.

The "C1-20 alkoxy group" of R¹ and R² refers to a group in which a linear or branched alkyl group having 1 to 20 carbon atoms is bonded to an oxygen atom. Examples include a methoxy group, ethoxy group, n-propoxy group, i-propoxy group, n-butoxy group, i-butoxy group, s-butoxy group, t-butoxy group, n-pentyloxy group, 1-ethylpropoxy group, n-hexyloxy group, isohexyloxy group, 4-methylpentoxy group, 3-methylpentoxy group, 2-methylpentoxy group, 1-methylpentoxy group, 3,3-dimethylbutoxy group, 2,2-dimethylbutoxy group, 1,1-dimethylbutoxy group, 1,2-dimethylbutoxy group, 1,3-dimethylbutoxy group, 2,3-dimethylbutoxy group, 1-ethylbutoxy group, 2-ethylbutoxy group, decyloxy group, dodecyloxy group, 2-ethyldecyloxy group and lauryloxy group. Among these, C1-6 alkoxy groups are preferable.

The "C1-20 haloalkoxy group" of R¹ and R² refers to a group in which a linear or branched haloalkyl group having 1 to 20 carbon atoms is bonded to an oxygen atom. Examples include a chloromethoxy group, dichloromethoxy group, trichloromethoxy group, trifluoromethoxy group, 1-fluoroethoxy group, 1,1-difluoroethoxy group, 2,2,2-trifluoroethoxy group, pentafluoroethoxy group, 4,5-dichloro-3-methylpentoxy group, 2-fluoro-2-ethyldecyloxy group and 1-chlorolauryloxy group. Among these, C1-6 haloalkoxy groups are preferable.

The "C3-20 cycloalkyl group" of R¹ and R² refers to an alkyl group having 3 to 20 carbon atoms having a cyclic moiety. Examples include a cyclopropyl group, cyclobutyl group, cyclopentyl group, cyclohexyl group, cycloheptyl group, cyclooctyl group, 2,3,3-trimethylcyclobutyl group, 4,4,6,6-tetramethylcyclohexyl group, 1,3-dibutylcyclohexyl group, norbornyl group, bicyclo[2.2.2]octyl group and adamantyl group. Among these, C3-6 cycloalkyl groups are preferable.

The "C4-20 cycloalkenyl group" of R¹ and R² refers to an alkenyl group having 4 to 20 carbon atoms having a cyclic moiety. Examples include a 1-cyclobutenyl group, 1-cyclopentenyl group, 3-cyclopentenyl group, 1-cyclohexenyl group, 3-cyclohexenyl group, 3-cycloheptenyl group, 4-cyclooctenyl group, 2-methyl-3-cyclohexenyl group and 3,4-dimethyl-3-cyclohexenyl group. Among these C4-8 cycloalkenyl groups are preferable.

The "C8-20 cycloalkynyl group" of R¹ and R² refers to an alkynyl group having 8 to 20 carbon atoms having a cyclic moiety. Examples include a 5-cyclooctynyl group, 6-cyclodecynyl group, 7-cyclododecynyl group and 2,3-diethyl-4-cyclodecynyl group. Among these C8-12 cycloalkynyl groups are preferable.

The "C1-20 acyl group" of R¹ and R² refers to a group in which a hydrogen atom, a linear or branched alkyl group having 1 to 19 carbon atoms, a linear or branched alkenyl group having 2 to 19 carbon atoms, a linear or branched alkynyl group having 2 to 19 carbon atoms, an aryl group having 1 to 19 carbon atoms or a heteroaryl group having 1 to 19 carbon atoms is bonded to a carbonyl group. Examples include a formyl group, acetyl group, propionyl group, n-propylcarbonyl group, i-propylcarbonyl group, n-butylcarbonyl group, i-butylcarbonyl group, pentanoyl group, pivaloyl group, valeryl group, isovaleryl group, octanoyl group, nonanoyl group, decanoyl group, 3-methylnonanoyl group, 8-methylnonanoyl group, 3-ethyloctanoyl group, 3,7-dimethyloctanoyl group, undecanoyl group, dodecanoyl group, tridecanoyl group, tetradecanoyl group, pentadecanoyl group, hexadecanoyl group, 1-methylpentadecanoyl group, 14-methylpentadecanoyl group, 13,13-dimethyltetradecanoyl group, heptadecanoyl group, 15-methylhexadecanoyl group, octadecanoyl group, 1-methylheptadecanoyl group, nonadecanoyl group, icosanoyl group, henaicosanoyl group, benzoyl group, naphthylcarbonyl group, biphenylcarbonyl group, anthranylcarbonyl group, 2-pyridylcarbonyl group and thienylcarbonyl group. Among these, C1-6 acyl groups are preferable.

The "C1-20 alkoxycarbonyl group" of R¹ and R² refers to a group in which a linear or branched alkoxy group having 1 to 20 carbon atoms is bonded to a carbonyl group. Examples include a methoxycarbonyl group, ethoxycarbonyl group, n-propoxycarbonyl group, i-propoxycarbonyl group, n-butoxycarbonyl group, i-butoxycarbonyl group, t-butoxycarbonyl group, n-pentyloxycarbonyl group, n-hexyloxycarbonyl group, decyloxycarbonyl group, dodecyloxycarbonyl group, 2-ethyldecyloxycarbonyl group and lauryloxycarbonyl group. Among these, C1-6 alkoxycarbonyl groups are preferable.

In addition, R¹ and R² may bond together to form an unsubstituted or substituted 5- to 8-membered ring. Examples of 5- to 8-membered rings include hydrocarbon rings and heterocycles, and hydrocarbon rings are preferable.
Examples of hydrocarbon rings include saturated hydrocarbon rings and unsaturated hydrocarbon rings, specific examples of saturated hydrocarbon rings include a cyclopentane ring, cyclohexane ring, cycloheptane ring and cyclooctane ring, while specific examples of unsaturated hydrocarbon rings include a cyclopentene ring, cyclohexene ring, cycloheptene ring and cyclooctene ring.
Examples of heterocycles include a tetrahydro-2H-pyran ring, tetrahydro-2H-thiopyran ring, piperidine ring, dioxane ring, oxathiane ring, morpholine ring, thiomorpholine ring, dithiane ring, piperazine ring, pyrrolidine ring, tetrahydrothiophene ring, tetrahydrofuran ring, 1,3-oxazolane ring, dithiolane ring, oxathiolane ring and dioxolane ring.
In addition, a condensed ring may be formed by condensing these rings with an optionally substituted aromatic hydrocarbon ring or aromatic hetero ring. Specific examples include a tetrahydronaphthalene ring, indane ring, tetrahydroisoquinoline ring, tetrahydrobenzofuran ring, 1,2-benzoisothiazole ring and cyclopentapyrimidine ring.

Specific examples of substituents when R¹ and R² have together bonded to form a 5- to 8-membered ring include a hydroxyl group; thiol group; cyano group; nitro group; halogen atoms such as a fluorine atom, chlorine atom or bromine atom; alkyl groups such as a methyl group, ethyl group, n-propyl group, i-propyl group or t-butyl group; alkenyl groups such as a vinyl group, 1-propenyl group, allyl group or 1-hexenyl group; alkynyl groups such as an ethynyl group, propargyl group, 3-pentynyl group or 1-hexynyl group; haloalkyl groups such as a chloromethyl group, trifluoromethyl group or perfluorohexyl group; haloalkenyl groups such as a 3-chloro-2-propenyl group, 3,3-difluoro-2-propenyl group or 2,4,6-trichloro-2-hexenyl group; haloalkynyl groups such as a 3-chloro-1-propynyl group, 3-chloro-1-butynyl group, 3-bromo-1-butynyl group or 3-bromo-2-propynyl group; alkoxy groups such as a methoxy group, ethoxy group, i-propoxy group or t-butoxy group; alkenyloxy groups such as a vinyloxy group, 1-propenyloxy group or allyloxy group; alkynyloxy groups such as a ethynyloxy group, propargyloxy group or 3-pentynyloxy group; haloalkoxy groups such as a chloromethoxy group, trifluoromethoxy group, 1-fluoroethoxy group or 1,1-difluoroethoxy group; haloalkenyloxy groups such as a 3-chloro-2-propenyloxy group, 4,4-difluoro-3-butenyloxy group or 3,3-difluoro-2-propenyloxy group; haloalkynyloxy groups such as a 3-chloro-1-butynyloxy group, 3-iodo-2-propynyloxy group or 5,5-dichloro-2-methyl-3-pentynyloxy group; alkylthio groups such as a methylthio group, i-propylthio group or t-butylthio group; alkenylthio groups such as a vinylthio group, 1-propenylthio group or allylthio group; alkynylthio groups such as a ethynylthio group, propargylthio group or 3-pentynylthio group; haloalkylthio groups such as a trifluoromethylthio group or 2,2-dichloroethylthio group; haloalkenylthio groups such as a 3-chloro-2-propenylthio group, 3,3-difluoro-2-propenylthio group or 2,4,6-trichloro-2-hexenylthio group; haloalkynylthio groups such as a 3-chloro-1-propynylthio group, 3-chloro-1-butynylthio group, 3-bromo-1-butynylthio group or 3-bromo-2-propynylthio group; alkylsulfonyl groups such as a methylsulfonyl group or i-propylsulfonyl group; alkenylsulfonyl groups such as a vinylsulfonyl group, 1-propenylsulfonyl group, allylsulfonyl group or 1-hexenylsulfonyl group; alkynylsulfonyl groups such as a ethynylsulfonyl group, propargylsulfonyl group, 3-pentynylsulfonyl group or 1-hexynylsulfonyl group; haloalkylsulfonyl groups such as a chloromethylsulfonyl group, trifluoromethylsulfonyl group or 2-bromoethylsulfonyl group; haloalkenylsulfonyl groups such as a 3,3-difluoro-2-propenylsulfonyl group or 2,4,6-trichloro-2-hexenylsulfonylgroup; haloalkynylsulfonyl groups such as a 3-chloro-1-propynylsulfonyl group, 3-chloro-1-butynylsulfonyl group or 3-bromo-1-butynylsulfonyl group; alkylsulfonyloxy groups such as an ethylsulfonyloxy group or t-butylsulfonyloxy group; alkenylsulfonyloxy groups such as a vinylsulfonyloxy group, 1-propenylsulfonyloxy group, allylsulfonyloxy group or 1-hexenylsulfonyloxy group; alkynylsulfonyloxy groups such as an ethynylsulfonyloxy group, propargylsulfonyloxy group, 3-pentynylsulfonyloxy group or 1-hexynylsulfonyloxy group; haloalkylsulfonyloxy groups such as a chloromethylsulfonyloxy group, trifluoromethylsulfonyloxy group or 2-bromoethylsulfonyloxy group; haloalkenylsulfonyloxy groups such as a 3-fluoro-2-propenylsulfonyloxy group or 3,3-difluoro-2-propenylsulfonyloxy group;
haloalkynylsulfonyloxy groups such as a 3-bromo-1-propynylsulfonyloxy group,
3-chloro-1-butynylsulfonyloxy group,
3-fluoro-1-butynylsulfonyloxy group or
3-bromo-2-propynylsulfonyloxy group; amino groups such as an amino group, methylamino group, ethylmethylamino group or phenylamino group; aryl groups such as a phenyl group or naphthyl group; and heterocyclic groups such as a pyridyl group, thienyl group, furyl group, pyridazinyl group or quinolyl group.

Among these, R¹ and R² each independently preferably represent a hydrogen atom, halogen atom, C1-20 alkyl group, C2-20 alkenyl group or C3-20 cycloalkyl group.

R³ to R⁶ each independently represent a hydrogen atom, halogen atom, C1-20 alkyl group, C1-20 haloalkyl group, C2-20 alkenyl group, C2-20 haloalkenyl group, C2-20 alkynyl group, C2-20 haloalkynyl group, C1-20 alkoxy group, C1-20 haloalkoxy group, C3-20 cycloalkyl group, C3-20 cycloalkoxy group, C2-20 alkenyloxy group, C2-20 alkynyloxy group, C1-20 alkylthio group, cyano group, nitro group, unsubstituted or substituted amino group, unsubstituted or substituted aryl group, unsubstituted or substituted aralkyl group, unsubstituted or substituted aryloxy group or unsubstituted or substituted heterocyclic group. R⁹ and R⁵, and R⁵ and R⁶ may bond together to form an unsubstituted or substituted 5- to 8-membered ring.

Specific examples of the halogen atom, C1-20 alkyl group, C1-20 haloalkyl group, C2-20 alkenyl group, C2-20 haloalkenyl group, C2-20 alkynyl group, C2-20 haloalkynyl group, C1-20 alkoxy group, C1-20 haloalkoxy group and C3-20 cycloalkyl group of R³ to R⁶ include the same groups as those listed as specific examples of R¹ and R².

The "C3-20 cycloalkoxy group" of R³ to R⁶ refers to a group in which a cycloalkyl group having 3 to 20 carbon atoms is bonded to an oxygen atom. Examples include a cyclopropyloxy group, cyclobutyloxy group, cyclopentyloxy group, cyclohexyloxy group, cycloheptyloxy group, cyclooctyloxy group, 2-methylcyclopropyloxy group, 2-ethylcyclopropyloxy group, 2,3,3-trimethylcyclobutyloxy group, 2-methylcyclopentyloxy group, 2-ethylcyclohexyloxy group, 2-ethylcyclooctyloxy group, 4,4,6,6-tetramethylcyclohexyloxy group and 1,3-dibutylcyclohexyloxy group. Among these, C3-6 cycloalkoxy groups are preferable.

The "C2-20 alkenyloxy group" of R³ to R⁶ refers to a group in which an alkenyl group having 2 to 20 carbon atoms is bonded to an oxygen atom. Examples include a vinyloxy group, 1-propenyloxy group, 2-propenyloxy group, 1-butenyloxy group, 2-butenyloxy group, 3-butenyloxy group,
1-methyl-2-propenyloxy group, 2-methyl-2-propenyloxy group, 1-pentenyloxy group, 2-pentenyloxy group, 3-pentenyloxy group, 4-pentenyloxy group, 1-methyl-2-butenyloxy group, 2-methyl-2-butenyloxy group, 1-hexenyloxy group, 2-hexenyloxy group, 3-hexenyloxy group, 4-hexenyloxy group, 5-hexenyloxy group, 1-heptenyloxy group, 6-heptenyloxy group, 1-octenyloxy group, 7-octenyloxy group, 1-decenyloxy group, 9-decenyloxy group, 1-dodecenyloxy group, 4-dodecenyloxy group, 1-octadecenyloxy group and 2-ethyl-1-octadecenyloxy group. Among these, C2-6 alkenyloxy groups are preferable.

The "C2-20 alkynyloxy group" of R³ to R⁶ refers to a group in which an alkynyl group having 2 to 20 carbon atoms is bonded to an oxygen atom. Examples include an ethynyloxy group, propynyloxy group, propargyloxy group, 1-butynyloxy group, 2-butynyloxy group, 3-butynyloxy group,
1-methyl-2-propynyloxy group, 2-methyl-3-butynyloxy group, 1-pentynyloxy group, 2-pentynyloxy group, 3-pentynyloxy group, 4-pentynyloxy group, 1-methyl-2-butynyloxy group, 2-methyl-3-pentynyloxy group, 1-hexynyloxy group, 1,1-dimethyl-2-butynyloxy group, dodecynyloxy group, butadecynyloxy group, heptadecynyloxy group and 4-ethylhexadecynyloxy group. Among these, C2-6 alkynyloxy groups are preferable.

The "C1-20 alkylthio group" of R³ to R⁶ refers to a group in which an alkyl group having 1 to 20 carbon atoms is bonded to a sulfur atom. Examples include a methylthio group, ethylthio group, n-propylthio group, i-propylthio group, n-butylthio group, t-butylthio group, pentylthio group, isopentylthio group, 2-methylbutylthio group, neopentylthio group, 1-ethylpropylthio group, hexylthio group, isohexylthio group, 4-methylpentylthio group, 3-methylpentylthio group, 2-methylpentylthio group, 1-methylpentylthio group, 3,3-dimethylbutylthio group, 2,2-dimethylbutylthio group, 1,1-dimethylbutylthio group, 1,2-dimethylbutylthio group, 1,3-dimethylbutylthio group, 2,3-dimethylbutylthio group, 2-ethylbutylthio group, 3-ethyloctylthio group, 2,3-dimethyloctylthio group, 4-propyloctylthio group, dodecylthio group, 4-ethyldecylthio group, 6-ethyldodecylthio group, 3,5-dimethyldecylthio group, 2,5-dimethyldecylthio group, 6-propylnonylthio group, 4-butyloctylthio group, tridecylthio group, tetradecylthio group,
2,4,6,8-tetramethyldecylthio group, 2,2-diethyldecylthio group, 2,5-diethyldecylthio group, 4-butyldecylthio group, pentadecylthio group, hexadecylthio group, heptadecylthio group, octadecylthio group, 2,4,6,8-tetraethyldecylthio group, nonadecylthio group and icosylthio group. Among these, C1-6 alkylthio groups are preferable.

Examples of "amino groups" of the "unsubstituted or substituted amino group" of R³ to R⁶ include an amino group; monoalkylamino groups such as a methylamino group or ethylamino group and preferably mono-C1-6 alkylamino groups; dialkylamino groups such as a dimethylamino group or diethylamino group and preferably di-C1-6 alkylamino groups; monoarylamino groups such as a phenylamino group or 4-methylphenylamino group and preferably mono-C6-10 arylamino groups; diarylamino groups such as a di-1-naphthylamino group and preferably di-C6-10 arylamino groups; and acylamino groups such as an acetylamino group or benzoylamino group and preferably C1-6 acylamino groups.

An "aryl group" of the "unsubstituted or substituted aryl group" of R³ to R⁶ refers to a monocyclic or polycyclic aryl group. Here, in the case of polycyclic aryl groups, partially saturated groups are also included in addition to completely unsaturated groups. Specific examples of aryl groups include a phenyl group, naphthyl group, azurenyl group, indenyl group, indanyl group and tetralinyl group. Among these, a phenyl group is preferable. Examples of substituents include the same substituents listed as specific examples of substituents when R¹ and R² bond together to form a 5- to 8-membered ring.

Specific examples of an "aralkyl group" of the "unsubstituted or substituted aralkyl group" of R³ to R⁶ include a benzyl group, phenethyl group, 3-phenylpropyl group, 1-naphthylmethyl group and 2-naphthylmethyl group. Among these, C6-10 aryl C1-6 alkyl groups are preferable. Examples of substituents include the same substituents listed as specific examples of substituents when R¹ and R² bond together to form a 5- to 8-membered ring.

An "aryloxy group" of the "unsubstituted or substituted aryloxy group" of R³ to R⁶ refers to a monocyclic or polycyclic aryloxy group. Here, in the case of a polycyclic aryloxy group, partially saturated groups are included in addition to completely unsaturated groups. Examples include a phenyloxy group, naphthyloxy group, azurenyloxy group, indenyloxy group, indanyloxy group and tetralinyloxy group. Among these, C6-10 aryloxy groups are preferable. Examples of substituents include the same substituents listed as specific examples of substituents when R¹ and R² bond together to form a 5- to 8-membered ring.

A "heterocyclic group" of the "unsubstituted or substituted heterocyclic group" of R³ to R⁶ refers to an aromatic heterocycle, saturated heterocycle, unsaturated heterocycle or condensed heterocycle in which these heterocycles are condensed with a benzene ring, that contain 1 to 4 hetero atoms other than carbon atoms selected from the group consisting of a nitrogen atom, oxygen atom and sulfur atom as atoms that compose the ring. Examples include a furan-2-yl group, furan-3-yl group, thiophen-2-yl group, thiophen-3-yl group, pyrrol-1-yl group, pyrrol-2-yl group, pyrrol-3-yl group, pyridin-2-yl group, pyridin-3-yl group, pyridin-4-yl group, pyrazin-2-yl group, pyrazin-3-yl group, pyrimidin-2-yl group, pyrimidin-4-yl group, pyrimidin-5-yl group, pyridazin-3-yl group, pyridazin-4-yl group, pyrrolidin-2-yl group, pyrrolidin-3-yl group, piperidin-2-yl group, piperidin-4-yl group, tetrahydrofuran-2-yl group, tetrahydrofuran-3-yl group, morpholin-2-yl group, morpholin-3-yl group, 1,3-benzodioxazol-4-yl group, 1,3-benzodioxazol-5-yl group, 1,4-benzodioxan-5-yl group, 1,4-benzodioxan-6-yl group, 3,4-dihydro-2H-1,5-benzodioxepin-6-yl group, 3,4-dihydro-2H-1,5-benzodioxepin-7-yl group, 2,3-dihydrobenzofuran-4-yl group, 2,3-dihydrobenzofuran-5-yl group, 2,3-dihydrobenzofuran-6-yl group,
2,3-dihydrobenzofuran-7-yl group, benzofuran-2-yl group, benzofuran-3-yl group, benzofuran-4-yl group, benzofuran-5-yl group, benzofuran-6-yl group, benzofuran-7-yl group, benzothiophen-2-yl group, benzothiophen-3-yl group, benzothiophen-4-yl group, benzothiophen-5-yl group, benzothiophen-6-yl group, benzothiophen-7-yl group, quinoxalin-2-yl group, quinoxalin-5-yl group, quinoxalin-6-yl group, indol-1-yl group, indol-2-yl group, indol-3-yl group, indol-4-yl group, indol-5-yl group, indol-6-yl group, indol-7-yl group, isoindol-1-yl group, isoindol-2-yl group, isoindol-4-yl group, isindol-5-yl group, isoindol-6-yl group, isoindol-7-yl group, isobenzofuran-1-yl group, isobenzofuran-4-yl group, isobenzofuran-5-yl group, isobenzofuran-6-yl group, isobenzofuran-7-yl group, chromen-2-yl group, chromen-3-yl group, chromen-4-yl group, chromen-5-yl group, chromen-6-yl group, chromen-7-yl group, chromen-8-yl group, imidazol-1-yl group, imidazol-2-yl group, imidazol-4-yl group, imidazol-5-yl group, pyrazol-1-yl group, pyrazol-3-yl group, pyrazol-4-yl group, pyrazol-5-yl group, thiazol-2-yl group, thiazol-4-yl group, thiazol-5-yl group, oxazol-2-yl group, oxazol-4-yl group, oxazol-5-yl group, isoxazol-3-yl group, isoxazol-4-yl group, isoxazol-5-yl group, benzoimidazol-1-yl group, benzoimidazol-2-yl group, benzoimidazol-4-yl group, benzoimidazol-5-yl group, benzothiazol-2-yl group, benzothiazol-4-yl group, benzothiazol-5-yl group, benzoxazol-2-yl group, benzoxazol-4-yl group, benzoxazol-5-yl group, quinolin-2-yl group, quinolin-3-yl group, quinolin-4-yl group, quinolin-5-yl group, quinolin-6-yl group, quinolin-7-yl group, quinolin-8-yl group, isoquinolin-1-yl group, isoquinolin-3-yl group, isoquinolin-4-yl group, isoquinolin-5-yl group, isoquinolin-6-yl group, isoquinolin-7-yl group, isoquinolin-8-yl group, 1,3,4-thiadiazol-2-yl group, morpholin-4-yl group, 1,2,3-triazol-1-yl group, 1,2,3-triazol-4-yl group, 1,2,3-triazol-5-yl group, 1,2,4-triazol-1-yl group, 1,2,4-triazol-3-yl group, 1,2,4-triazol-5-yl group, tetrazol-1-yl group and tetrazol-2-yl group. Among these, 5- to 10-membered heterocyclic groups are preferable. Examples of substituents include the same substituents listed as specific examples of substituents when R¹ and R² bond together to form a 5- to 8-membered ring.

Among these, R³ to R⁶ are preferably halogen atoms, C1-20 alkyl groups, C1-20 haloalkyl groups, C3-20 cycloalkyl groups, cyano groups, nitro groups, or unsubstituted or substituted heterocyclic groups.

In addition, R⁴ and R⁵, and R⁵ and R⁶ may bond together to form an unsubstituted or substituted 5- to 8-membered ring. More specifically, examples include the same as those listed as example of rings formed by R¹ and R², or they may form an aromatic ring, an aromatic hydrocarbon ring or an aromatic heterocycle.
Examples of an aromatic hydrocarbon ring include a benzene ring, examples of an aromatic heterocycle include a furan ring, thiophene ring, pyrrole ring, pyridine ring, pyrazine ring, pyrimidine ring, pyridazine ring, imidazole ring, pyrazole ring, thiazole ring, oxazole ring and isoxazole ring.
Among these, cases in which R⁵ and R⁶ form a ring are preferable, and the ring is preferably an unsubstituted or substituted benzene ring. Examples of substituents include the same substituents listed as specific examples of substituents when R¹ and R² bond together to form a 5- to 8-membered ring, and a halogen atom, hydroxyl group, C1-4 alkyl group or C1-4 alkoxy group is preferable.

R⁷ represents a hydrogen atom, halogen atom, hydroxyl group, C1-20 alkyl group, C1-20 haloalkyl group, C2-20 alkenyl group, C2-20 haloalkenyl group, C2-20 alkynyl group, C2-20 haloalkynyl group, C1-20 alkoxy group, C1-20 haloalkoxy group, C3-20 cycloalkyl group, C3-20 cycloalkoxy group, C2-20 alkenyloxy group, C2-20 alkynyloxy group, C1-20 alkylthio group or cyano group.

Specific examples of the halogen atom, C1-20 alkyl group, C1-20 haloalkyl group, C2-20 alkenyl group, C2-20 haloalkenyl group, C2-20 alkynyl group, C2-20 haloalkynyl group, C1-20 alkoxy group, C1-20 haloalkoxy group, C3-20 cycloalkyl group, C3-20 cycloalkoxy group, C2-20 alkenyloxy group, C2-20 alkynyloxy group and C1-20 alkylthio group of R⁷ include the same as those listed as specific examples of the aforementioned R¹ to R⁶.

X represents a halogen atom, nitro group, unsubstituted or substituted amino group, hydroxyl group or organic group.
Examples of the halogen atom and unsubstituted or substituted amino group of X include the same as those listed as examples of R¹ to R⁶.

There are no particular limitations on the organic group of X provided it is an atomic group that contains carbon atoms. Examples include a cyano group; isocyano group; cyanato group; isocyanato group; thiocyanato group; isothiocyanato group; carboxyl group; and the C1-20 alkyl group, C1-20 haloalkyl group, C2-20 alkenyl group, C2-20 haloalkenyl group, C2-20 alkynyl group, C2-20 haloalkynyl group, C1-20 alkoxy group, C1-20 haloalkoxy group, C3-20 cycloalkyl group, C4-20 cycloalkenyl group, C8-20 cycloalkynyl group, C1-20 acyl group, C1-20 alkoxycarbonyl group, C3-20 cycloalkoxy group, C2-20 alkenyloxy group, C2-20 alkynyloxy group, C1-20alkylthio group, unsubstituted or substituted aryl group, unsubstituted or substituted aralkyl group, unsubstituted or substituted aryloxy group and unsubstituted or substituted heterocyclic group listed as examples of the aforementioned R¹ to R⁶.

Among these, X is preferably a halogen atom, alkyl group or haloalkyl group.
n represents an integer of 0 to 4, and is preferably 0, 1 or 2.

Z represents an oxygen atom, sulfur atom, or group represented by the formula: CR⁸R⁹ or the formula: NR¹⁰.
R⁸ and R⁹ each independently represent a hydrogen atom, halogen atom, C1-20 alkyl group, C1-20 haloalkyl group, C2-20 alkenyl group, C2-20 haloalkenyl group, C2-20 alkynyl group, C2-20 haloalkynyl group, C1-20 alkoxy group, C1-20 haloalkoxy group, C3-20 cycloalkyl group, C4-20 cycloalkenyl group, C8-20 cycloalkynyl group, C1-20 acyl group or C1-20 alkoxycarbonyl group. R⁸ and R⁹ may bond together to form an unsubstituted or substituted 5- to 8-membered ring.

Specific examples of the halogen atom, C1-20 alkyl group, C1-20 haloalkyl group, C2-20 alkenyl group, C2-20 haloalkenyl group, C2-20 alkynyl group, C2-20 haloalkynyl group, C1-20 alkoxy group, C1-20 haloalkoxy group, C3-20 cycloalkyl group, C4-20 cycloalkenyl group, C8-20 cycloalkynyl group, C1-20 acyl group and C1-20 alkoxycarbonyl group of R⁸ and R⁹ are the same as those listed as specific examples of the aforementioned R¹ to R².

R⁸ and R⁹ may bond together to form an unsubstituted or substituted 5- to 8-membered ring. More specifically, examples include the same as those listed as examples of unsubstituted or substituted 5- to 8-membered rings formed together by R¹ and R², and a saturated hydrocarbon ring is preferable.

R¹⁰ represents a hydrogen atom, C1-20 alkyl group, C2-20 alkenyl group, C2-20 alkynyl group, C1-20 acyl group, C1-20 alkoxycarbonyl group, C1-20 alkylsulfonyl group, C1-20 alkoxy C1-20 alkyl group, C2-20 acyloxy C1-20 alkyl group, unsubstituted or substituted aminocarbonyl group, unsubstituted or substituted arylsulfonyl group or unsubstituted or substituted aminosulfonyl group.

Specific examples of the C1-20 alkyl group, C2-20 alkenyl group, C2-20 alkynyl group, C1-20 acyl group and C1-20 alkoxycarbonyl group of R¹⁰ are the same as those listed as specific examples of R¹ and R².

Specific examples of the C1-20 alkylsulfonyl group of R¹⁰ include a methylsulfonyl group, ethylsulfonyl group, n-propylsulfonyl group, i-propylsulfonyl group, n-butylsulfonyl group, isobutylsulfonyl group, sec-butylsulfonyl group, tert-butylsulfonyl group, n-pentylsulfonyl group, isopentylsulfonyl group, neopentylsulfonyl group, 1-ethylpropylsulfonyl group, n-hexylsulfonyl group, isohexylsulfonyl group, 4-methylpentylsulfonyl group, 3-methylpentylsulfonyl group, 2-methylpentylsulfonyl group, 1-methylpentylsulfonyl group, 3,3-dimethylbutylsulfonyl group, 2,2-dimethylbutylsulfonyl group, 1,1-dimethylbutylsulfonyl group,
1,2-dimethylbutylsulfonyl group, 1,3-dimethylbutylsulfonyl group, 2,3-dimethylbutylsulfonyl group, 1-ethylbutylsulfonyl group and 2-ethylbutylsulfonyl group. Among these, C1-6 alkylsulfonyl groups are preferable.

The "C1-20 alkoxy C1-20 alkyl group" of R¹⁰ refers to a group in which a C1-20 alkoxy group and a C1-20 alkyl group are bonded. Specific examples include a methoxymethyl group, ethoxymethyl group, methoxyethyl group, ethoxyethyl group, methoxy-n-propyl group, ethoxymethyl group, ethoxyethyl group, n-propoxymethyl group, i-propoxyethyl group, s-butoxymethyl group and t-butoxyethyl group. Among these, C1-6 alkoxy C1-6 alkyl groups are preferable.

The "C2-20 acyloxy C1-20 alkyl group" of R¹⁰ refers to a group in which a C2-20 acyloxy group and C1-20 alkyl group are bonded. Specific examples include a acetoxymethyl group, 2-acetoxyethyl group, propionyloxymethyl group, propionyloxyethyl group, benzoyloxymethyl group and benzoyloxyethyl group. Among these, C2-7 acyloxy C1-6 alkyl groups are preferable.

Specific examples of the unsubstituted or substituted aminocarbonyl group of R¹⁰ include aminocarbonyl groups; monoalkylaminocarbonyl groups such as a methyaminocarbonyl group or ethylaminocarbonyl group, and preferably mono-C1-6 alkylaminocarbonyl groups; dialkylaminocarbonyl groups such as a dimethylaminocarbonyl group or ethylmethylaminocarbonyl group, and preferably di-C1-6 alkylaminocarbonyl groups; monoarylaminocarbonyl groups such as a phenylaminocarbonyl group or 4-methylphenylaminocarbonyl group and preferably mono-C6-10 arylaminocarbonyl groups; diarylaminocarbonyl groups such as a diphenylaminocarbonyl group or di-1-naphthylaminocarbonyl group and preferably di-C6-10 arylaminocarbonyl groups; and acylaminocarbonyl groups such as an acetylaminocarbonyl group or benzoylaminocarbonyl group and preferably C1-6 acylaminocarbonyl groups.

Specific examples of the unsubstituted or substituted arylsulfonyl group of R¹⁰ include a phenylsulfonyl group, 4-methylphenylsulfonyl group, 2,6-dichlorophenylsulfonyl group, 2-nitrophenylsulfonyl group and 1-naphthylsulfonyl group. Among these, C6-10 arylsulfonyl groups are preferable.

Specific examples of the unsubstituted or substituted aminosulfonyl group of R¹⁰ include an aminosulfonyl group; monoalkylaminosulfonyl groups such as a methylaminosulfonyl group or ethylaminosulfonyl group and preferably mono-C1-6 alkylaminosulfonyl groups; dialkylaminosulfonyl groups such as a dimethylaminosulfonyl group or ethylmethylaminosulfonyl group and preferably di-C1-6 alkylaminosulfonyl groups; monoarylaminosulfonyl groups such as a phenylaminosulfonyl group or 4-methylphenylaminosulfonyl group and preferably mono-C6-10 arylaminosulfonyl groups; diarylaminosulfonyl groups such as a diphenylaminosulfonyl group or di-1-naphthylaminosulfonyl group and preferably di-C6-10 arylaminosulfonyl groups; and acylaminosulfonyl groups such as an acetylaminosulfonyl group or benzoylaminosulfonyl group and preferably C1-C6 acylaminosulfonyl groups.

Among these, Z is preferably an oxygen atom, sulfur atom, or group represented by the formula: CR^{8'}R^{9'} (wherein, R^{8'} and R^{9'} each independently represent a hydrogen atom, methyl group or ethyl group) or the formula: NR^{10'} (wherein, R^{10'} represents a hydrogen atom, methyl group or ethyl group).

### (Production Method)

The nitrogen-containing heterocyclic compound of the present invention can be produced by a known method, or can be produced, for example, in the manner described below.
A compound represented by formula (II):

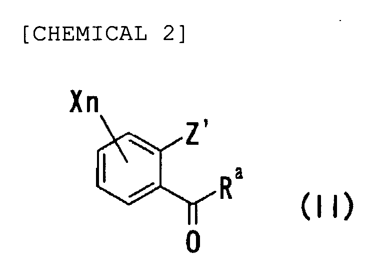

(wherein, X and n are the same as previously defined in formula (I) , R^{a} represents a hydrogen atom or esterified hydroxyl group, and Z' represents a substituent able to derived to Z) is reacted with a compound represented by formula (III):

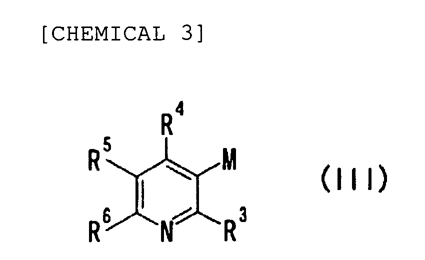

(wherein, R³ to R⁶ are the same as previously defined in formula (I), and M represents a leaving group) to be able to obtain an alcohol compound represented by formula (IV):

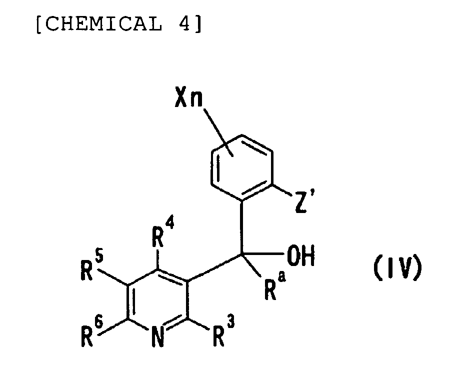

(wherein, R³ to R⁶, X, n, R^{a} and Z' are the same as previously defined in formulas (I) and (II)).
In formula (II), examples of the esterified hydroxyl group represented by R^{a} preferably include hydroxyl groups esterified with a methyl group or ethyl group.
The substituent represented by Z' that is able to be derived to Z can be arbitrarily selected corresponding to the derived reaction. In addition, Z' and R^{a} may bond together to form a cyclic structure.
In formula (III), examples of the leaving group represented by M include Li, Na, K, Ca(1/2), MgCl, MgBr, MgI, ZnCl, SnCl, Sn(n-Bu)₃, CrCl₂, CuCl, CuBr, NiCl, PdCl and B(OH)₂.

The reaction is carried out at a temperature range of about -50 to 50°C in an inert solvent such as tetrahydrofuran, diethyl ether, dimethoxyethane, hexane, toluene, benzene, methylene chloride, chloroform, 1, 2-dichloroethane, DMF, DMSO or a mixed solvent thereof. In addition, the reaction is preferably carried out in an inert gas (such as nitrogen or argon) atmosphere. The reaction can also be allowed to proceed smoothly by adding a catalyst such as
tetrakis(triphenylphosphine)palladium (0) or tetrakis(triphenylphosphine)nickel (0) as necessary.

A compound represented by formula (II) serving as the starting material is either a known compound or can be produced according to a method indicated in the examples.

Moreover, X and R¹ to R⁶ in a compound represented by formula (I) can be converted to other substituents by the known method indicated below. For example, a hydrogen atom can be converted to a halogen atom by halogenation, to a nitro group by nitration, to an amino group by reduction of a nitro group, or can be derived to an acylamino group or sulfonylamino group by acylation or sulfonation of an amino group. A cyano group can be converted to a carbamoyl group by, for example, treated with aqueous sodium hydroxide/30% aqueous hydrogen peroxide, or to a thiocarbamoyl group by using hydrogen sulfide in pyridine/triethylamine. In addition, a cyano group can also be converted to a carboxyl group by, for example, heating and hydrolyzing in an aqueous sodium hydroxide, or to a formyl group by using Raney nickel in water/acetic acid/pyridine in the presence of sodium phosphate. A formyl group can be converted to a vinyl group by a Wittig reaction, or to a hydroxyiminomethyl group, for example, by reacting with a hydroxyamine. A hydroxyl group can be converted to an alkoxy group by alkylation or to an acyloxy group by acylation, and an alkylhydroxyl group can be converted to a nitroxyalkyl group by sulfuric acid/nitric acid.

In the case Z' is an amino group or hydroxyl group, the alcohol form represented by formula (IV) can be used to produce a compound represented by formula (I) by carrying out a cyclization reaction by reacting the alcohol form with a compound represented by formula (V):

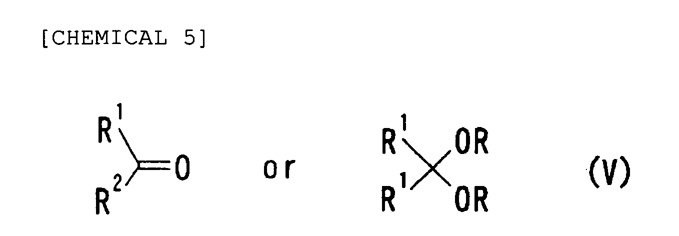

(wherein, R¹ and R² are the same as previously defined in formula (I), and R represents a methyl group or ethyl group) in the presence of an acid catalyst.

A ring closing reaction between an alcohol form represented by formula (IV) and a compound represented by formula (V) is carried out at a temperature range of room temperature to 100°C in the absence of a solvent, or in the presence of an inert solvent such as benzene, toluene, chloroform, dichloromethane, 1,2-dichloroethane, diethyl ether or a mixed solvent thereof, and in the presence of an acid catalyst and dehydrating agent. Examples of acid catalysts that can be used include hydrochloric acid, p-toluenesulfonic acid, benzenesulfonic acid, camphorsulfonic acid, ammonium chloride and ammonium acetate. The amount of acid catalyst used is preferably suitably altered according to the degree of acidity of the catalyst, and is preferably within the range of about 1/100 to 10 times the number of moles of the alcohol form represented by formula (IV). Examples of dehydrating agents that can be used include a molecular sieve, anhydrous calcium sulfate and anhydrous magnesium sulfate.

In addition, an alcohol represented by formula (IV) can be used to produce a compound represented by formula (I) by deriving to a compound represented by formula (IV'):

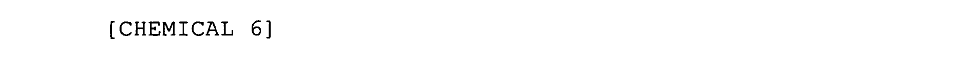

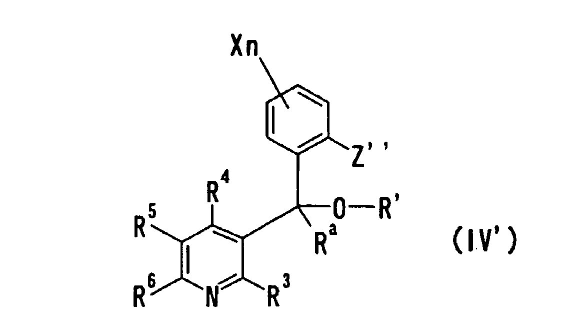

(wherein, R³ to R⁶, X, n and R^{a} are the same as previously defined in formula (IV), Z" represents a halogen atom, and R' represents a substituted allyl group) in accordance with established methods, and carrying out a cyclization reaction in the presence of a palladium catalyst.

Examples of the "allyl group" of R' include a 2-methyl-allyl group and 2-methyl-but-2-en-1-yl group.

Among compounds represented by formula (I), a basic compound can be converted to a salt using an acid. A suitable acid for this reaction is an acid that yields an agriculturally and horticulturally acceptable salt. Examples include inorganic acids such as hydrochloric acid, hydrobromic acid, phosphoric acid, sulfuric acid, nitric acid or sulfamic acid, and organic acids such as acetic acid, tartaric acid, citric acid, fumaric acid, maleic acid, p-toluenesulfonic acid, methanesulfonic acid or glutamic acid.
A salt of a compound represented by formula (I) can be produced by, for example, allowing an inorganic acid or organic acid to act on a compound represented by formula (I).

In each of these reactions, the target compound can be isolated following completion of the reaction by commonly known purification means such as distillation, recrystallization or column chromatography if purification of the product is required following an ordinary post-treatment procedure.

The structure of a target compound can be identified and confirmed by known analysis means such as elementary analysis, NMR spectroscopy, IR spectroscopy or mass spectrometry.

Examples of compounds of the present invention obtained in the manner described above are shown in the following Tables 1 to 8.
In the tables, Me represents a methyl group, Et represents an ethyl group, n-Pr represents an n-propyl group, cPr represents a cyclopropyl group, Ph represents a phenyl group, and Bn represents a benzyl group.
In addition, when R⁵ and R⁶ bond together to form a phenyl (Ph) group, substitution sites are indicated as quinoline. In addition, when R¹ and R² bond together to form a ring, the left side indicates bonding with R¹ while the right side indicates bonding with R². Similarly, when R⁴ and R⁵ bond together to form a ring, the left side indicates bonding with R⁴ while the right side indicates bonding with R⁵. When R⁵ and R⁶ bond together to form a ring other than a phenyl group, the left side indicates bonding with R⁵ while the right side indicates bonding with R⁶.

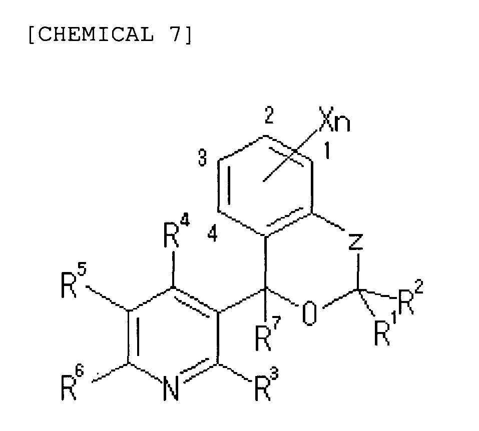

| Compound No. | R¹ | R² | R³ | R⁴ | R⁵ | R⁶ | R⁷ | Xn | Z |
|---|---|---|---|---|---|---|---|---|---|
| 1-1 | H | H | H | H | Ph | | H | - | O |
| 1-2 | H | Me | H | H | Ph | | H | - | O |
| 1-3 | Me | Me | H | H | Ph | | H | - | O |
| 1-4 | Et | Me | H | H | Ph | | H | - | O |
| 1-5 | n-Pr | Me | H | H | Ph | | H | - | O |
| 1-6 | CH₂=CH | Me | H | H | Ph | | H | - | O |
| 1-7 | Br | Me | H | H | Ph | | H | - | O |
| 1-6 | Me O | Me | H | H | Ph | | H | - | O |
| 1-9 | Me | Me | H | Me | Ph | | H | - | O |
| 1-10 | Me | CF₃ | H | Et | Ph | | H | - | O |
| 1-11 | Me | Me | H | n-Pr | Ph | | H | - | O |
| 1-12 | Me | Me | H | CH₂=CH | Ph | | H | - | O |
| 1-13 | Me | Me | H | Br | Ph | | H | - | O |
| 1-14 | Me | Me | H | Me O | Ph | | H | - | O |
| 1-15 | Me | Me | H | CF₃ | Ph | | H | - | O |
| 1-16 | Me | Me | H | CN | Ph | | H | - | O |
| 1-17 | Me | Me | H | NO₂ | Ph | | H | - | O |
| 1-18 | H | H | Cl | H | Ph | | H | - | O |
| 1-19 | H | Me | Cl | H | Ph | | H | - | O |
| 1-20 | H | Et | Cl | H | Ph | | H | - | O |
| 1-21 | H | n-Pr | Cl | H | Ph | | H | - | O |
| 1-22 | H | CH₂=CH | Cl | H | Ph | | H | - | O |
| 1-23 | H | Cl | Cl | H | Ph | | H | - | O |
| 1-24 | H | MeO | Cl | H | Ph | | H | - | O |
| 1-25 | H | Me | H | H | 5-F-Ph | | H | - | O |
| 1-26 | H | Me | H | H | 6-FPh | | H | - | O |
| 1-27 | H | Me | H | H | 7-Cl-Ph | | H | - | O |
| 1-28 | H | Me | H | H | 8-Cl-Ph | | H | - | O |
| 1-29 | H | Me | H | H | 5-Me-Ph | | H | - | O |
| 1-30 | H | Me | H | H | 6-Me-Ph | | H | - | O |
| 1-31 | H | Me | H | H | 6-MeO-Ph | | H | - | O |
| 1-32 | H | Me | H | H | 7-MeO-Ph | | H | - | O |
| 1-33 | H | Me | H | H | 5-OH-Ph | | H | - | O |

| Compound No. | R¹ | R² | R³ | R⁴ | R⁵ | R⁶ | R⁷ | Xn | Z |
|---|---|---|---|---|---|---|---|---|---|
| 1-34 | H | Me | H | H | 6-OH-Ph | | F | - | O |
| 1-35 | Me | Me | H | H | Me | C I | H | - | O |
| 1-36 | H | Me | H | H | H | Et | CN - | | O |
| 1-37 | H | Me | H | H | H | Me O | H | - | O |
| 1-38 | H | Me | H | H | H | EtO | H | - | O |
| 1-39 | H | Me | H | H | H | NO₂ | H | - | O |
| 1-40 | H | Me | H | H | H | CN | H | - | O |
| 1-41 | H | Me | H | H | H | NH₂ | H | - | O |
| 1-42 | H | Me | H | H | H | F | H | - | O |
| 1-43 | H | Me | H | Bn | H | Br | H | - | O |
| 1-44 | H | Me | H | cPr | H | CF₃ | H | - | O |
| 1-45 | Me | Me | H | H | -(CH₂)₄- | | H | - | O |
| 1-46 | -(CH₂)₅- | | H | H | Ph | | H | - | O |
| 1-47 | -(CH₂)₅- | | Me | Me | Ph | | H | - | O |
| 1-48 | -(CH₂)₅- | | Et | Et | Ph | | H | - | O |
| 1-49 | -(CH₂)₅- | | MeO | MeO | Ph | | H | - | O |
| 1-50 | Me | Me | H | H | -CH=CH-S- | | H | - | O |
| 1-51 | -(CH₂)₄- | | H | H | Ph | | H | - | O |

| Compound No. | R¹ | R² | R³ | R⁴ | R⁵ | R⁶ | R⁷ | Xn | Z |
|---|---|---|---|---|---|---|---|---|---|
| 2-1 | H | H | H | H | Ph | | H | 1-F | O |
| 2-2 | H | Me | H | H | Ph | | H | 1-F | O |
| 2-3 | Me | Me | H | H | Ph | | H | 1-F | O |
| 2-4 | CH₂=CH | Me | H | H | Ph | | H | 1-F | O |
| 2-5 | Br | Me | H | H | Ph | | H | 1-F | O |
| 2-6 | MeO | Me | H | H | Ph | | H | 1-F | O |
| 2-7 | Me | Et | H | H | Ph | | H | 1-F | O |
| 2-8 | Me | Me | H | CH₂=CH | Ph | | H | 1-F | O |
| 2-9 | Me | Me | H | Br | Ph | | H | 1-F | O |
| 2-10 | Me | Me | H | MeO | Ph | | H | 1-F | O |
| 2-11 | Me | Me | H | CF₃ | Ph | | H | 1-F | O |
| 2-12 | Me | Me | H | C N | Ph | | H | 1-F | O |
| 2-13 | Me | Me | H | NO₂ | Ph | | H | 1-F | O |
| 2-14 | H | H | Cl | H | Ph | | H | 1-F | O |
| 2-15 | H | Me | Cl | H | Ph | | H | 1-F | O |
| 2-16 | H | Cl | Cl | H | Ph | | H | 1-F | O |
| 2-17 | Me | Me | H | H | 8-F-Ph | | H | 1-F | O |
| 2-18 | H | Me | H | H | 6-Me-Ph | | H | 1-F | O |
| 2-19 | H | Me | H | H | 6-MeO-Ph | | H | 1-F | O |
| 2-20 | H | Me | H | H | H | Me | H | 1-F | O |
| 2-21 | H | Me | H | H | H | MeO | H | 1-F | O |
| 2-22 | H | Me | H | H | H | F | H | 1-F | O |
| 2-23 | H | Me | H | H | H | CF₃ | H | 1-F | O |
| 2-24 | H | MeO | H | Cl | Ph | | H | 1-F | O |
| 2-25 | -(CH₂)₅- | | H | H | Ph | | H | 1 -F | O |
| 2-26 | -(CH₂)₅- | | Me | Me | Ph | | H | 1-F | O |
| 2-27 | -(CH₂)₅- | | MeO | MeO | Ph | | H | 1-F | O |
| 2-28 | -(CH₂)₅- | | H | H | 6-F-Ph | | H | 1-F | O |
| 2-29 | -(CH₂)₅- | | H | H | 6-Me-Ph | | H | 1-F | O |
| 2-30 | -(CH₂)₅- | | H | H | 6-MeO-Ph | | H | 1-F | O |
| 2-31 | Et | Et | H | H | Ph | | H | 1-F | O |
| 2-32 | Me | Me | H | H | Ph | | H | 1-Cl | O |

| Compound No. | R¹ | R² | R³ | R⁴ | R⁵ | R⁶ | R⁷ | Xn | Z |
|---|---|---|---|---|---|---|---|---|---|
| 2-33 | Me | Me | H | H | 8-F-Ph | | H | 1-Cl | O |
| 2-34 | Me | Me | H | MeO | Ph | | H | 1-Cl | O |
| 2-35 | Me | Me | H | CF₃ | Ph | | H | 1-Cl | O |
| 2-36 | Me | Me | H | CN | Ph | | H | 1-Cl | O |
| 2-37 | Me | Me | H | NO₂ | Ph | | H | 1-Cl | O |
| 2-38 | -(CH₂)₄- | | H | H | Ph | | H | 1-Cl | O |
| 2-39 | -(CH₂)₄- | | Me | Me | Ph | | H | 3-Cl | O |
| 2-40 | -(CH₂)₄- | | MeO | MeO | Ph | | H | 4-Br | O |
| 2-41 | Me | Me | H | H | Ph | | H | 1 ,2-F₂ | O |
| 2-42 | Me | Me | H | Me | Ph | | H | 1 ,2-F₂ | O |
| 2-43 | Me | Me | H | MeO | Ph | | H | 1,2-F₂ | O |
| 2-44 | Me | Me | H | CF₃ | Ph | | H | 1 ,2-F₂ | O |
| 2-45 | Me | Me | H | CN | Ph | | H | 1,2-F₂ | O |
| 2-46 | Me | Me | H | NO₂ | Ph | | H | 1,2-F₂ | O |
| 2-47 | -(CH₂)₄- | | H | H | Ph | | H | 1,2-F₂ | O |
| 2-48 | -(CH₂)₄- | | Me | Me | Ph | | H | 1,2-F₂ | O |
| 2-49 | -(CH₂)₄- | | MeO | MeO | Ph | | H | 1,2-F₂ | O |

| Compound No. | R¹ | R² | R³ | R⁴ | R⁵ | R⁶ | R⁷ | Xn | Z |
|---|---|---|---|---|---|---|---|---|---|
| 3-1 | H | H | H | H | Ph | | H | 2-NO₂ | S |
| 3-2 | H | Me | H | H | Ph | | H | 1-OH | S |
| 3-3 | Me | Me | H | H | Ph | | H | 1-Me | S |
| 3,4 | CH₂=CH | Me | H | H | Ph | | H | 3-NH₂ | S |
| 3-5 | Br | Me | H | H | Ph | | H | 2-Ph | S |
| 3-6 | MeO | Me | H | H | Ph | | H | - | S |
| 3-7 | Me | Me | H | Me | Ph | | H | - | S |
| 3-8 | Me | Me | H | CH₂=CH | Ph | | H | - | S |
| 3-9 | Me | Me | H | Br | Ph | | H | - | S |
| S-10 | Me | Me | H | MeO | Ph | | H | - | S |
| 3-11 | Me | Me | H | CF₃ | Ph | | H | - | S |
| 3-12 | Me | Me | H | CN | Ph | | H | - | S |
| 3-13 | Me | Me | H | NO₂ | Ph | | H | - | S |
| 3-14 | H | H | Cl | H | Ph | | H | - | S |
| 3-15 | H | Me | Cl | H | Ph | | H | - | S |
| 3-16 | H | Cl | Cl | H | Ph | | H | - | S |
| 3-17 | H | Me | H | H | 6-F-Ph | | H | - | S |
| 3-18 | H | Me | H | H | 6-Me-Ph | | H | - | S |
| 3-19 | H | Me | H | H | 6-MeO-Ph | | H | - | S |
| 3-20 | H | Me | H | H | H | Me | H | - | S |
| 3-21 | H | Me | H | H | H | Me O | H | - | S |
| 3-22 | H | Me | H | H | H | F | H | - | S |
| 3-23 | H | Me | H | H | H | CF₃ | H | - | S |
| 3-24 | H | MeO | H | C I | Ph | | H | - | S |
| 3-25 | -(CH₂)₅- | | H | H | Ph | | H | - | S |
| 3-26 | -(CH₂)₅- | | Me | Me | Ph | | H | - | S |
| 3-27 | -(CH₂)₅- | | MeO | MeO | Ph | | H | - | S |
| 3-28 | -(CH₂)₅- | | H | H | 6-F-Ph | | H | - | S |
| 3-29 | -(CH₂)₅- | | H | H | 6-Me-Ph | | H | - | S |
| 3-30 | -(CH₂)₅- | | H | H | 6-MeO-Ph | | H | - | S |
| 3-31 | Et | Et | H | H | Ph | | H | - | S |

| Compound No. | R¹ | R² | R³ | R⁴ | R⁵ | R⁶ | R⁷ | Xn | Z |
|---|---|---|---|---|---|---|---|---|---|
| 3-32 | Me | Me | H | H | Ph | | H | 1-F | S |
| 3-33 | Me | Me | H | Me | Ph | | H | 1-F | S |
| 3-34 | Me | Me | H | MeO | Ph | | H | 1-F | S |
| 3-35 | Me | Me | H | CF₃ | Ph | | H | 1-F | S |
| 3-36 | Me | Me | H | CN | Ph | | H | 1-F | S |
| 3-37 | Me | Me | H | NO₂ | Ph | | H | 1-F | S |
| 3-38 | -(CH₂)₄- | | H | H | Ph | | H | 1-F | S |
| 3-39 | -(CH₂)₄- | | Me | Me | Ph | | H | 1-F | S |
| 3-40 | -(CH₂)₄- | | MeO | MeO | Ph | | H | 1-F | S |
| 3-41 | Me | Me | H | H | Ph | | H | 1,2-F₂ | S |
| 3-42 | Me | Me | H | Me | Ph | | H | 1,2-F₂ | S |
| 3-43 | Me | Me | H | MeO | Ph | | H | 1,2-F₂ | S |
| 3-44 | Me | Me | H | CF₃ | Ph | | H | 1,2-F₂ | S |
| 3-45 | Me | Me | H | CN | Ph | | H | 1,2-F₂ | S |
| 3-46 | Me | Me | H | NO₂ | Ph | | H | 1 ,2-F₂ | S |
| 3-47 | -(CH₂)₄- | | H | H | Ph | | H | 1 ,2-F₂ | S |
| 3-48 | -(CH₂)₄- | | Me | Me | Ph | | H | 1 ,2-F₂ | S |
| 3-49 | -(CH₂)₄- | | MeO | MeO | Ph | | H | 1 ,2-F₂ | S |

| Compound No. | R¹ | R² | R³ | R⁴ | R⁵ | R⁶ | R⁷ | Xn | Z |
|---|---|---|---|---|---|---|---|---|---|
| 4-1 | H | H | H | H | Ph | | H | - | NH |
| 4-2 | H | Me | H | H | Ph | | H | - | NH |
| 4-3 | Me | Me | H | H | Ph | | H | - | NH |
| 4-4 | CH₂=CH | Me | H | H | Ph | | H | - | NH |
| 4-5 | Br | Me | H | H | Ph | | H | - | NH |
| 4-6 | MeO | Me | H | H | Ph | | H | - | NH |
| 4-7 | Me | Me | H | Me | Ph | | H | - | NH |
| 4-8 | Me | Me | H | CH₂=CH | Ph | | H | - | NH |
| 4-9 | Me | Me | H | Br | Ph | | H | - | NH |
| 4-10 | Me | Me | H | MeO | Ph | | H | - | NH |
| 4-11 | Me | Me | H | CF₃ | Ph | | H | - | NH |
| 4-12 | Me | Me | H | CN | Ph | | H | - | NH |
| 4-13 | Me | Me | H | NO₂ | Ph | | H | - | NH |
| 4-14 | H | H | Cl | H | Ph | | H | - | NH |
| 4-15 | H | Me | Cl | H | Ph | | H | - | NH |
| 4-16 | H | Cl | Cl | H | Ph | | H | - | NH |
| 4-17 | H | Me | H | H | 6-F-Ph | | H | - | NH |
| 4-18 | H | Me | H | H | 6-Me-Ph | | H | - | NH |
| 4-19 | H | Me | H | H | 6-MeO-Ph | | H | - | NH |
| 4-20 | H | Me | H | H | H | Me | H | - | NH |
| 4-21 | H | Me | H | H | H | MeO | H | - | NH |
| 4-22 | H | Me | H | H | H | F | H | - | NH |
| 4-23 | H | Me | H | H | H | CF₃ | H | - | NH |
| 4-24 | H | MeO | H | Cl | Ph | | H | - | NH |
| 4-25 | -(CH₂)₅- | | H | H | Ph | | H | - | NH |
| 4-26 | -(CH₂)₅- | | Me | Me | Ph | | H | - | NH |
| 4-27 | -(CH₂)₅- | | MeO | MeO | Ph | | H | - | NH |
| 4-28 | -(CH₂)₅- | | H | H | 6-F-Ph | | H | - | NH |
| 4-29 | -(CH₂)₅- | | H | H | 6-Me-Ph | | H | - | NH |
| 4-30 | -(CH₂)₅- | | H | H | 6-MeO-Ph | | H | - | NH |
| 4-31 | Et | Et | H | H | Ph | | H | - | NH |

| Compound No. | R¹ | R² | R³ | R⁴ | R⁵ | R⁶ | R⁷ | Xn | Z |
|---|---|---|---|---|---|---|---|---|---|
| 4-32 | Me | Me | H | H | Ph | | H | 1-F | NH |
| 4-33 | Me | Me | H | Me | Ph | | H | 1-F | NH |
| 4-34 | Me | Me | H | MeO | Ph | | H | 1 -F | NH |
| 4-35 | Me | Me | H | CF₃ | Ph | | H | 1 -F | NH |
| 4-36 | Me | Me | H | CN | Ph | | H | 1 -F | NH |
| 4-37 | Me | Me | H | NO₂ | Ph | | H | 1-F | NH |
| 4-38 | -(CH₂)₄- | | H | H | Ph | | H | 1 -F | NH |
| 4-39 | -(CH₂)₄- | | Me | Me | Ph | | H | 1-F | NH |
| 4-40 | -(CH₂)₄- | | MeO | MeO | Ph | | H | 1-F | NH |
| 4-41 | Me | Me | H | H | Ph | | H | 1,2-F₂ | NH |
| 4-42 | Me | Me | H | Me | Ph | | H | 1,2-F₂ | NH |
| 4-43 | Me | Me | H | MeO | Ph | | H | 1,2-F₂ | NH |
| 4,44 | Me | Me | H | CF₃ | Ph | | H | 1,2-F₂ | NH |
| 4-45 | Me | Me | H | CN | Ph | | H | 1,2-F₂ | NH |
| 4-46 | Me | Me | H | NO₂ | Ph | | H | 1,2-F₂ | NH |
| 4-47 | -(CH₂)₄- | | H | H | Ph | | H | 1,2-F₂ | NH |
| 4-48 | -(CH₂)₄- | | Me | Me | Ph | | H | 1,2-F₂ | NH |
| 4-49 | -(CH₂)₄- | | MeO | MeO | Ph | | H | 1,2-F₂ | NH |

| Compound No. | R¹ | R² | R³ | R⁴ | R⁵ | R⁶ | R⁷ | Xn | Z |
|---|---|---|---|---|---|---|---|---|---|
| 5-1 | H | H | H | H | Ph | | H | - | NMe |
| 5-2 | H | Me | H | H | Ph | | H | - | NMe |
| 5-3 | Me | Me | H | H | Ph | | H | - | NMe |
| 5-4 | CH₂=CH | Me | H | H | Ph | | H | - | NMe |
| 5-5 | Br | Me | H | H | Ph | | H | - | NMe |
| 5-6 | MeO | Me | H | H | Ph | | H | - | NMe |
| 5-7 | Me | | H | Me | Ph | | H | - | NMe |
| 5-8 | Me | Me | H | CH₂=CH | Ph | | H | - | NMe |
| 5-9 | Me | Me | H | Br | Ph | | H | - | NMe |
| 5-10 | Me | Me | H | MeO | Ph | | H | - | NMe |
| 5-11 | Me | Me | H | CF₃ | Ph | | H | - | NMe |
| 5-12 | Me | Me | H | CN | Ph | | H | - | NMe |
| 5-13 | Me | Me | H | NO₂ | Ph | | H | - | NMe |
| 5-14 | H | H | Cl | H | Ph | | H | - | NMe |
| 5-15 | H | Me | Cl | H | Ph | | H | - | NMe |
| 5-16 | H | Cl | Cl | H | Ph | | H | - | NMe |
| 5-17 | H | Me | H | H | 6-F-Ph | | H | - | NMe |
| 5-18 | H | Me | H | H | 6-Me-Ph | | H | - | NMe |
| 5-19 | H | Me | H | H | 6-MeO-Ph | | H | - | NMe |
| 5-20 | H | Me | H | H | H | Me | H | - | NMe |
| 5-21 | H | Me | H | H | H | MeO | H | - | NMe |
| 5-22 | H | Me | H | H | H | F | H | - | NMe |
| 5-23 | H | Me | H | H | H | CF₃ | H | - | NMe |
| 5-24 | H | MeO | H | Cl | Ph | | H | - | NMe |
| 5-25 | -(CH₂)₅- | | H | H | Ph | | H | - | NMe |
| 5-26 | -(CH₂)₅- | | Me | Me | Ph | | H | - | NMe |
| 5-27 | --(CH₂)₅- | | MeO | MeO | Ph | | H | - | NMe |
| 5-28 | -(CH₂)₅- | | H | H | 6-F-Ph | | H | - | NMe |
| 5-29 | -(CH₂)₅- | | H | H | 6-Me-Ph | | H | - | NMe |
| 5-30 | -(CH₂)₅- | | H | H | 6-MeO-Ph | | H | - | NMe |
| 5-31 | Et | Et | H | H | Ph | | H | - | NMe |

| Compound No. | R¹ | R² | R³ | R⁴ | R⁵ | R⁶ | R⁷ | Xn | Z |
|---|---|---|---|---|---|---|---|---|---|
| 5-32 | Me | Me | H | H | Ph | | H | 1-F | NMe |
| 5-33 | Me | Me | H | Me | Ph | | H | 1-F | NMe |
| 5-34 | Me | Me | H | MeO | Ph | | H | 1-F | NMe |
| 5-35 | Me | Me | H | CF₃ | Ph | | H | 1-F | NMe |
| 5-36 | Me | Me | H | CN | Ph | | H | 1-F | NMe |
| 5-37 | Me | Me | H | NO₂ | Ph | | H | 1-F | NMe |
| 5-38 | -(CH₂)₄- | | H | H | Ph | | H | 1-F | NMe |
| 5-39 | -(CH₂)₄- | | Me | Me | Ph | | H | 1-F | NMe |
| 5-40 | -(CH₂)₄- | | MeO | MeO | Ph | | H | 1-F | NMe |
| 5-41 | Me | Me | H | H | Ph | | H | 1,2-F₂ | NMe |
| 5-42 | Me | Me | H | Me | Ph | | H | 1,2-F₂ | NMe |
| 5-43 | Me | Me | H | MeO | Ph | | H | 1,2-F₂ | NMe |
| 5-44 | Me | Me | H | CF₃ | Ph | | H | 1,2-F₂ | NMe |
| 5-45 | Me | Me | H | CN | Ph | | H | 1,2-F₂ | NMe |
| 5-46 | Me | Me | H | NO₂ | Ph | | H | 1,2-F₂ | NMe |
| 5-47 | -(CH₂)₄- | | H | H | Ph | | H | 1,2-F₂ | NMe |
| 5-48 | -(CH₂)₄- | | Me | Me | Ph | | H | 1,2-F₂ | NMe |
| 5-49 | -(CH₂)₄- | | MeO | MeO | Ph | | H | 1,2-F₂ | NMe |

| Compoud No. | R¹ | R² | R³ | R⁴ | R⁵ | R⁶ | R⁷ | Xn | Z |
|---|---|---|---|---|---|---|---|---|---|
| 6-1 | H | H | H | H | Ph | | H | - | CH₂ |
| 6-2 | H | Me | H | H | Ph | | H | - | CH₂ |
| 6-3 | Me | Me | H | H | Ph | | H | - | CH₂ |
| 6-4 | CH₂=CH | Me | H | H | Ph | | H | - | CH₂ |
| 6-5 | Me | Me | H | Me | Ph | | H | - | CH₂ |
| 6-6 | Me | Me | H | CH₂=CH | Ph | | H | - | CH₂ |
| 6-7 | Me | Me | H | Br | Ph | | H | - | CH₂ |
| 6-8 | Me | Me | H | MeO | Ph | | H | - | CH₂ |
| 6-9 | Me | Me | H | CF₃ | Ph | | H | - | CH₂ |
| 6-10 | Me | Me | H | CN | Ph | | H | - | CH₂ |
| 6-11 | Me | Me | H | NO₂ | Ph | | H | - | CH₂ |
| 6-12 | H | H | Cl | H | Ph | | H | - | CH₂ |
| 6-13 | Me | Me | Cl | H | Ph | | H | - | CH₂ |
| 6-14 | Me | Me | H | H | 8-F-Ph | | H | - | CH₂ |
| 6-15 | Me | Me | H | H | 6-Me-Ph | | H | - | CH₂ |
| 6-16 | Me | Me | H | H | 6-MeO-Ph | | H | - | CH₂ |
| 6-17 | Me | Me | H | H | H | Me | H | - | CH₂ |
| 6-18 | Me | Me | H | H | H | MeO | H | - | CH₂ |
| 6-19 | Me | Me | H | H | H | F | H | - | CH₂ |
| 6-20 | Me | Me | H | H | H | CF₃ | H | - | CH₂ |
| 6-21 | Et | Et | H | H | Ph | | H | - | CH₂ |
| 6-22 | Me | Me | H | H | Ph | | H | 1-F | CH₂ |
| 6-23 | Me | Me | H | Me | Ph | | H | 1-F | CH₂ |
| 6-24 | Me | Me | H | MeO | Ph | | H | 1-F | CH₂ |
| 6-25 | Me | Me | H | CF₃ | Ph | | H | 1-F | CH₂ |
| 6-26 | Me | Me | H | CN | Ph | | H | 1-F | CH₂ |
| 6-27 | Me | Me | H | NO₂ | Ph | | H | 1-F | CH₂ |
| 6-28 | Me | Me | H | H | 8-F-Ph | | H | 1-F | CH₂ |
| 6-29 | Me | Me | H | Me | Ph | | H | 1,2-F₂ | CH₂ |
| 6-30 | Me | Me | H | MeO | Ph | | H | 1,2-F₂ | CH₂ |
| 6-31 | Me | Me | H | CF₂ | Ph | | H | 1,2-F₂ | CH₂ |
| 6-32 | Me | Me | H | CN | Ph | | H | 1,2-F₂ | CH₂ |
| 6-33 | Me | Me | H | NO₂ | Ph | | H | 1,2-F₂ | CH₂ |

| Compound No. | R¹ | R² | R³ | R⁴ | R⁵ | R⁶ | R⁷ | Xn | Z |
|---|---|---|---|---|---|---|---|---|---|
| 7-1 | H | H | H | H | Ph | | H | - | C(Me)₂ |
| 7-2 | H | Me | H | H | Ph | | H | - | C(Me)₂ |
| 7-3 | H | H | H | Me | Ph | | H | - | C(Me)₂ |
| 7-4 | H | H | H | CH₂=CH | Ph | | H | - | C(Me)₂ |
| 7-5 | H | H | H | Br | Ph | | H | - | C(Me)₂ |
| 7-6 | H | H | H | MeO | Ph | | H | - | C(Me)₂ |
| 7-7 | H | H | H | CF₃ | Ph | | H | - | C(Me)₂ |
| 7-8 | H | H | H | CN | Ph | | H | - | C(Me)₂ |
| 7-9 | H | H | H | NO₂ | Ph | | H | - | C(Me)₂ |
| 7-10 | H | H | Cl | H | Ph | | H | - | C(Me)₂ |
| 7-11 | H | Me | Cl | H | Ph | | H | - | C(Me)₂ |
| 7-12 | H | H | H | H | 6-F-Ph | | H | - | C(Me)₂ |
| 7-13 | H | H | H | H | 6-Me-Ph | | H | - | C(Me)₂ |
| 7-14 | H | H | H | H | 6-MeO-Ph | | H | - | C(Me)₂ |
| 7-15 | H | H | H | H | H | Me | H | - | C(Me)₂ |
| 7-16 | H | H | H | H | H | MeO | H | - | C(Me)₂ |
| 7-17 | H | H | H | H | H | F | H | - | C(Me)₂ |
| 7-18 | H | H | H | H | H | CF₃ | H | - | C(Me)₂ |
| 7-19 | H | H | H | H | Ph | | H | 1-F | C(Me)₂ |
| 7-20 | H | H | H | Me | Ph | | H | 1-F | C(Me)₂ |
| 7-21 | H | H | H | MeO | Ph | | H | 1-F | C(Me)₂ |
| 7-22 | H | H | H | CF₃ | Ph | | H | 1-F | C(Me)₂ |
| 7-23 | H | H | H | CN | Ph | | H | 1-F | C(Me)₂ |
| 7-24 | H | H | H | NO₂ | Ph | | H | 1 -F | C(Me)₂ |
| 7-25 | H | H | H | H | Ph | | H | 1,2-F₂ | C(Me)₂ |
| 7-26 | H | H | H | Me | Ph | | H | 1,2-F₂ | C(Me)₂ |
| 7-27 | H | H | H | MeO | Ph | | H | 1,2-F₂ | C(Me)₂ |
| 7-28 | H | H | H | CF₃ | Ph | | H | 1,2-F₂ | C(Me)₂ |
| 7-29 | H | H | H | CN | Ph | | H | 1,2-F₂ | C(Me)₂ |
| 7-30 | H | H | H | N0₂ | Ph | | H | 1,2-F₂ | C(Me)₂ |

| Compound No. | R¹ | R² | R³ | R⁴ | R⁵ | R⁶ | R⁷ | Xn | Z |
|---|---|---|---|---|---|---|---|---|---|
| 8-1 | H | H | H | H | Ph | | OH | - | CH₂ |
| 8-2 | H | Me | H | H | Ph | | OH | - | CH₂ |
| 8-3 | Me | Me | H | H | Ph | | OH | - | CH₂ |
| 8-4 | Me | Me | H | Me | Ph | | OH | - | CH₂ |
| 8-5 | Me | Me | H | CH₂=CH | Ph | | OH | - | CH₂ |
| 8-6 | Me | Me | H | Br | Ph | | OH | - | CH₂ |
| 8-7 | Me | Me | H | MeO | Ph | | OH | - | CH₂ |
| 8-8 | Me | Me | H | CF₃ | Ph | | OH | - | CH₂ |
| 8-9 | Me | Me | H | CN | Ph | | OH | - | CH₂ |
| 8-10 | Me | Me | H | NO₂ | Ph | | OH | - | CH₂ |
| 8-11 | Me | Me | H | H | 6-F-Ph | | OH | - | CH₂ |
| 8-12 | Me | Me | H | H | 6-Me-Ph | | OH | - | CH₂ |
| 8-13 | Me | Me | H | H | 6-MeO-Ph | | OH | - | CH₂ |
| 8-14 | Me | Me | H | H | H | Me | OH | - | CH₂ |
| 8-15 | Et | Et | H | H | Ph | | OH | - | CH₂ |
| 8-16 | Me | Me | H | H | Ph | | OH | 1-F | CH₂ |
| 8-17 | Me | Me | H | H | Ph | | OH | 1,2-F₂ | CH₂ |
| 8-18 | H | H | H | H | Ph | | MeO | - | CH₂ |
| 8-19 | H | Me | H | H | Ph | | MeO | - | CH₂ |
| 8-20 | Me | Me | H | H | Ph | | MeO | - | CH₂ |
| 8-21 | Me | Me | H | Me | Ph | | MeO | - | CH₂ |
| 8-22 | Me | Me | H | CH₂=CH | Ph | | MeO | - | CH₂ |
| 8-23 | Me | Me | H | Br | Ph | | MeO | - | CH₂ |
| 8-24 | Me | Me | H | MeO | Ph | | MeO | - | CH₂ |
| 8-25 | Me | Me | H | CF₃ | Ph | | MeO | - | CH₂ |
| 8-26 | Me | Me | H | CN | Ph | | MeO | - | CH₂ |
| 8-27 | Me | Me | H | NO₂ | Ph | | MeO | - | CH₂ |
| 8-28 | Me | Me | H | H | 6-F-Ph | | MeO | - | CH₂ |
| 8-29 | Me | Me | H | H | 6-Me-Ph | | MeO | - | CH₂ |
| 8-30 | Me | Me | H | H | 6-MeO-Ph | | MeO | - | CH₂ |
| 8-31 | Me | Me | H | H | H | Me | MeO | - | CH₂ |
| 8-32 | Et | Et | H | H | Ph | | MeO | - | CH₂ |
| 8-33 | Me | Me | H | H | Ph | | MeO | 1-F | CH₂ |
| 8-34 | Me | Me | H | H | Ph | | MeO | 1,2-F₂ | CH₂ |

### 2) Fungicide for Agricultural and Horticultural Use

A nitrogen-containing heterocyclic compound of the present invention represented by formula (I), or a salt thereof (to be referred to as "the compound of the present invention") can be used as a fungicide for agricultural and horticultural use (to be referred to as "the fungcide of the present invention") that contains at least one thereof as an active ingredient thereof.

The amount of active ingredient is normally 0.01 to 90% by weight and preferably 0.05 to 85% by weight based on the total weight of the fungicide.

The fungicide of the present invention demonstrates superior bactericidal action against a wide range of types of fungi, such as fungi belonging to Oomycetes, Ascomycetes Deuteromycetes and Basidiomycetes.

A composition having the fungicide of the present invention as an active ingredient thereof can be used to control various plant diseases occurring during cultivation of agricultural and horticultural crops such as flowering plants, lawn grasses and pasture grasses by seed treatment, foliar spraying, soil application or water surface application and the like.

Examples of crops in which plant diseases can be controlled along with their plant diseases and causative organisms include:
Sugar Beets:
   Cercospora leaf spot (Cercospora beticola)
Peanuts:
   Brown leaf spot (Mycosphaerella arachidis)
   Black leaf blight (Mycosphaerella berkeleyi)
Cucumbers:
   Powdery mildew (Sphaerotheca fuliginea)
   Gummy stem blight (Mycosphaerella melonis)
   Sclerotinia rot (Sclerotinia sclerotiorum)
   Gray mold (Botrytis cinerea)
   Scab (Cladosporium cucumerinum)
   Corynespora leaf spot (Corynespora cassicola)
   Damping-off (Pythium debaryanam, Rhizoctonia solani Kuhn)
   Bacterial spot (Pseudomonas syringae pv. Lecrymans)
Tomatoes:
   Gray mold (Botrytis cinerea)
   Leaf mold (Cladosporium fulvum)
Eggplants:
   Gray mold (Botrytis cinerea)
   Black rot (Corynespora malongenae)
   Powdery mildew (Erysiphe cichoracearum)
   Leaf mold (Mycovelloslella nattrassii)
Strawberries:
   Gray mold (Botrytis cinerea)
   Powdery mildew (Sphaerotheca humuli)
   Anthracnose (Colletotrichum acutatum, Colletotrichum fragariae)
Onions:
   Neck rot (Botrytis allii)
   Gray mold (Botrytis cinerea)
   Leaf blight (Botrytis squamosa)
Cabbage:
   Clubroot (Plasmodiophora brassicae)
   Bacterial soft rot (Erwinia carotovora)
Kidney beans:
   Stem rot (Sclerotinia sclerotiorum)
   Gray mold (Botrytis cinerea)
Apples:
   Powdery mildew (Podosphaera leucotricha)
   Scab (Venturia inaequalis)
   Blossom blight (Monilinia mali)
   Valsa canker (Valsa mali)
   Alternaria blotch (Alternaria mali)
   Rust (Gymnosporangium yamadae)
   Ring rot (Botryosphaeria berengeriana)
   Anthracnose (Coletotrichum gloeosprioides)
   Blotch (Diplocarpon mali)
Persimmons:
   Powdery mildew (Phyllactinia kakicola)
   Anthracnose (Gloeosporium kaki)
   Angular leaf spot (Cercospora kaki)

Peaches and cherries:
   Brown rot (Monilinia fructicola)
Grapes:
   Gray mold (Botrytis cinerea)
   Powdery mildew (Uncinula necator)
   Ripe rot (Glomerella cingulata)
Pears:
   Scab (Venturia nashicola)
   Rust (Gymnosporangium asiaticum)
   Black spot (Alternaria kikuchiana)
Tea:
   Gray blight (Pestalotia theae)
   Anthracnose (Collectotrichum theae-sinensis)
Citrus:
   Scab (Elsinoe fawcette)
   Blue mold (Penicillium italicum)
   Common green mold (Penicillium digitatum)
   Gray mold (Botrytis cinerea)
   Melanose (Diaporthe citri)
   Canker (Xanthomonas campestris pv. Citri)
Wheat:
   Powdery mildew (Erysiphe graminis f. sp. tritici)
   Fusarium blight (Gibberella zeae)
   Leaf rust (Puccinia recondita)
   Browining root rot (Pythium iwayamai)
   Snow mold (Monographella nivalis)
   Eye spot (Pseudocercosporella herpotrichoides)
   Speckled leaf blotch (Septoria tritici)
   Glume blotch (Leptosphaeria nodorum)
   Typhula snow blight (Typhula incarnata)
   Sclerotinia snow blight (Myriosclerotinia borealis)
   Take-all (Gaeumanomyces graminis)

Barley:
   Stripe (Pyrenophora graminia)
   Leaf blotch (Rhynchosporium secalis)
   Loose smut (Ustilago tritici, U. nuda)
Rice:
   Blast (Pyricularia oryzae)
   Sheath blight (Rhizoctonia solani)
   Bakanae disease (Gibberella fujikuroi)
   Brown spot (Cochliobolus niyabeanus)
   Seedling blight (Pythium graminicolum)
Tobacco:
   Sclerotinia stem-rot (Sclerotinia sclerotiorum)
   Powdery mildew (Erysiphe cichoracearum)
Tulips:
   Gray mold (Botrytis cinerea)
Bent grass:
   Sclerotinia snow blight (Sclerotinia borealis)
   Bacterial shoot blight (Pythium aphanidermatum)
Orchard grass:
   Powdery mildew (Erysiphe graminis)
Soybeans:
   Purple stain (Cercospora kikuchii)
   Downy mildew (Peronospora manshurica)
   Phytophthora root and stem rot (Phytophthora sojae)
Potatoes, tomatoes:
   Late blight (Phytophthora infestans)
Cucumbers:
   Downy mildew (Pseudoperonospora cubensis)
Grapes:
   Downy mildew (Plasmopara viticola)

In addition, various pathogens have recently developed resistance to benzimidazole fungicides and dicarboximide fungicides resulting in inadequate efficacy of these drugs, thereby creating the need for effective drugs against resistant organisms as well. The fungicide of the present invention also has superior bactericidal effects against resistant organisms in addition to pathogens that are sensitive to these drugs.

For example, the fungicide of the present invention is effective against gray mold (Botrytis cinerea), sugar beet cercospora leaf spot (Cercospora beticola) and apple scab (Venturia inaequalis), pear scab (Venturia nashicola), which exhibit resistance to benzimidazole fungicides such as thiophanate-methyl, benomyl, carbendazim and thiabendazole, in the same manner as sensitive organisms.

Moreover, the fungicide of the present invention is effective against gray mold (Botrytis cinerea), which exhibits resistance to dicarboximide fungicides (such as vinclozoline, procymidone and iprodione) in the same manner as sensitive organisms.

Examples of diseases for which application of the fungicide of the present invention is more preferable include sugar beet cercospora leaf spot, wheat powdery mildew, rice blast, apple scab, cucumber gray mold and peanut brown leaf spot.

In addition, the fungicide of the present invention causes little chemical damage, exhibits low toxicity to fish and warm-blooded animals, and has a high degree of safety.
The fungicide of the present invention may be used in the pure form of a compound of the present invention without adding other components, or may be used in a form able to be adopted to an ordinary agricultural chemical, such as a wettable powder, granules, powder, emulsion, aqueous solution, suspension or water-dispersible granules, by using an additive or carrier.

Examples of additives and carriers able to be added to the agricultural chemical preparation used for the purpose of solid formulations include vegetable powders such as soybean powder or wheat powder, mineral fine powders such as diatomaceous earth, apatite, gypsum, talc, bentonite, pyrophyllite or clay, and organic and inorganic compounds such as sodium benzoate, urea or sodium sulfate.

In addition, in the case of using for the purpose of liquid formulations, kerosene, xylene and petroleum-based aromatic hydrocarbons, cyclohexane, cyclohexanone, dimethylformamide, dimethylsulfoxide, alcohol, acetone, trichloroethylene, methyl isobutyl ketone, mineral oil, vegetable oil and water, for example, can be used as solvents.

Moreover, a surfactant can be added to these preparations as necessary to obtain a uniform and stable form.
There are no particular limitations on surfactants used, and examples include nonionic surfactants such as polyoxyethylene-alkyl phenyl ethers, polyoxyethylene-alkyl ethers, polyoxyethylene-higher fatty acid esters, polyoxyethylene-sorbitan fatty acid esters or polyoxyethylene-tristyryl phenyl ether, and sulfuric acid ester salts of polyoxyethylene-alkyl phenyl ethers, alkyl benzene sulfonates, sulfuric acid ester salts of higher alcohols, alkyl naphthalene sulfonates, polycarboxylates, lignin sulfonates, formaldehyde condensates of alkyl naphthalene sulfonates and isobutylene-maleic anhydrate copolymers.

Wettable powders, emulsions, flowable agents, aqueous solutions and water-dispersible granules are used in the form of solutions, suspensions or emulsions by diluting to a prescribed concentration with water, while powders and granules are used by spraying directly onto plants.

A formulated fungicide of the present invention is applied to a plant body, seeds, water surface or soil either as is or after diluting with water and the like. Although the applied amount varies according to weather conditions, preparation form, application time, application method, applied location, target control disease, target crop and the like, it is normally 1 to 1,000 g and preferably 10 to 100 g as the amount of active ingredient compound per hectare.

In the case of applying by diluting a wettable powder, emulsion, suspension, aqueous solution or water-dispersible granules with water, the applied concentration is 1 to 1000 ppm and preferably 10 to 250 ppm, while in the case of granules or powder, it can also be applied as is without diluting.

In addition to the compound of the present invention, the fungicide of the present invention can also be mixed with one type or two or more types of various fungicides, insecticides, miticides or synergists.

Typical examples of fungicides, insecticides, miticides and plant growth regulators able to be used by mixing with the compound of the present invention are indicated below.

Fungicides:
   captan, folpet, thiuram, ziram, zineb, maneb, mancozeb, propineb, polycarbamate, chlorothalonil, quintozene, captafol, iprodione, procymidone, vinclozolin, fluoroimide, cymoxanil, mepronil, flutolanil, pencycuron, oxycarboxin, fosetyl-aluminum, propamocarb, triadimefon, triadimenol, propiconazole,diclobutorazol, bitertanol, hexaconazol, miclobutanil, flusilazole, metconazole, etaconazole, fluotrimazole, cyproconazole, epoxyconazole, flutriafen, penconazole, diniconazole, cyproconazole, fenarimol, triflumizole, prochloraz, imazalil, pefurazoate, tridemorph, fenpropimorph, triforine, buthiobate, pyrifenox, anilazine, polyoxin, metalaxyl, oxadixyl, furalaxyl, isoprothiolane, probenazole, pyrrolnitrin, blasticidin S, kasugamycin, validamycin, dihydrostreptomycin sulfate, benomyl, carbendazim, thiophanate-methyl, hymexazol, basic copper chloride, basic copper sulfate, fentin acetate, triphenyltin hydroxide, diethofencarb, methasulfocarb, qinomethionate, binapacryl, lecithin, sodium bicarbonate, dithianon, dinocap, fenaminosulf, diclomezine, guazatine, dodine, IBP, edifenphos, mepanipyrim, ferimzone, trichlamide, methasulfocarb, fluazinam, ethoqinolac, dimethomorph, pyroquilon, tecloftalam, fthalide, phenazine oxide, thiabendazole, tricyclazole, vinclozolin, cymoxanil, cyclobutanil, guazatine, propamocarb hydrochloride, oxolinic acid, hydroxyisoxazole, iminoctadine acetate, and the like.

Insecticides/Miticides:
   Organic phosphorous and carbamate-based insecticides: fenthion, fenitrothion, diazinon, chlorpyrifos, ESP, vamidothion, phenthoate, dimethoate, formothion, malathion, trichlorfon, thiometon, phosmet, dichlorvos, acephate, EPBP, methyl parathion, oxydemetone methyl, ethion, salithion, cyanophos, isoxathion, pyridafenthion, phosalone, methidathion, sulprofos, chlorfenvinphos, tetrachlorovinphos, dimethylvinphos, propaphos, isofenphos, ethyl thiometon, profenofos, pyraclofos, monocrotophos, azinphos-methyl, aldicarb, methomyl, thiodicarb, carbofuran, carbosulphan, benfuracarb, furathiocarb, propoxur, BPMC, MTMC, MIPC, carbaryl, pirimicarb, ethiophencarb, phenoxycarb, EDDP, and the like.
      Pyrethroid-based insecticides: permethrin, cypermethrin, deltamethrin, fenvalerate, fenpropathrin, pyrethrin, allethrin, tetramethrin, resmethrin, dimethrin, propathrin, phenothrin, prothrin, fluvalinate, cyfluthrin, cyhalothrin, flucythrinate, etofenprox, cycloprothrin, tralomethrin, silafluofen, flufenprox, acrinathrin, and the like. Benzoylurea-based and other insecticides: diflubenzuron, chlorfluazuron, hexaflumuron, triflumuron, tetrabenzuron, flufenoxuron, flucycloxuron, buprofezin, pyriproxyfen, methoprene, benzoepin, diafenthiuron, acetamiprid, imidacloprid, nitenpyram, fipronil, cartap, thiocyclam, bensultap, nicotine sulfate, rotenone, metaldehyde, machine oil, BT and microbial agrichemicals such as insect pathogenic viruses.

Nematocides:
   Fenamiphos, fosthiazate, and the like
Miticides:
   Chlorobenzilate, phenisobromolate, dicofol, amitraz, BPPS, benzomate, hexythiazox, fenbutatin oxide, polynactin, chinomethionate, CPCBS, tetradifon, avermectin, milbemectin, clofentezine, cyhexatin, pyridaben, fenpyroximate, tebufenpyrad, pyrimidifen, fenothiocarb, dienochlor, and the like.
Plant growth regulators:
   Gibberellins (such as gibberellin A3, gibberellin A4 or gibberellin A7), IAA, NAA.

### Examples

The following provides a more detailed explanation of the present invention by indicating examples thereof. However, the present invention is not limited to the following examples.

### [Example 1]

### Production of 1,2-dihydro-2,2-dimethyl-4-(3-quinolyl) -4H-3,1-benzoxazine

p-toluenesufonate monohydrate (13.5 mg, 0.071 mmol) was added to a toluene (5 mL)-acetone (1 mL) solution of (2-aminophenyl)-quinolin-3-yl-methanol (0.18 g, 0.71 mmol) followed by stirring for 24 hours at room temperature. Subsequently, the reaction solution was poured into a mixed solution of diethyl ether (50 mL), saturated aqueous sodium bicarbonate solution (20 mL) and water (30 mL), and the separated aqueous phase was extracted with ethyl acetate (50 mL). The combined organic phase was washed with brine (50 mL) and dried with magnesium sulfate. After filtering, the solvent was distilled off under reduced pressure and the resulting residue was purified by silica gel column chromatography (first pass: silica gel 80 g, n-hexane:ethyl acetate = 8:1 to 4:1, second pass: NH-silica gel 50 g, n-hexane : ethyl acetate = 4:1) to obtain 0.02 g (10%) of the target compound.
Physical properties: mp 122 to 124°C
¹H NMR (300 MHz, CDCl₃): δ 1.58 (s,3H), 1.61 (s, 1H), 6.05 (s, 1H), 6.57-6.72 (m, 3H), 7.09 (m, 1H), 7.54 (m, 1H), 7.71 (m, 1H), 7.81 (d, J=8.7 Hz, 1H), 8.11 (d, J=8.7Hz, 1H), 8.16 (d, J=2.1 Hz, 1H), 8.88 (d, J=2.1 Hz, 1H); MS (APCI, m/z): 291 ([M+1] +)

### [Example 2]

### Production of 1,2-dihydro-1,2,2-trimethyl-4-(3-quinolyl) -4H-3,1-benzoxazine

NaH (40.9 mg, 1. 02 mmol) was added to a DMF (5 mL) solution of 1,2-dihydro-2,2-dimethyl-4-(3-quinolyl)-4H-3,1-benzoxazine (0.27 g) followed by stirring for 30 minutes at room temperature. Subsequently, MeI (0.197 g, 1.39 mmol) was added followed by stirring for 1 hour at room temperature. Moreover, NaH (40.9 mg, 1. 02 mmol) and MeI (0.197g, 1.39mmol) were further added followed by stirring for 2 hours at room temperature. The reaction solution was poured into a mixed solution of ethyl acetate (50 mL), saturated aqueous ammonium chloride solution (30 mL) and water (30 mL), and the separated aqueous phase was extracted with ethyl acetate (50 mL). The combined organic phase was washed with brine (30 mL) and dried with magnesium sulfate. After filtering, the solvent was distilled off under reduced pressure and the resulting residue was purified by silica gel column chromatography (NH-silica gel 80 g, n-hexane:ethyl acetate = 8:1 to 4:1) to obtain 0.05 g (18%) of the target compound.
Physical properties: amorphous
¹HNMR (300 MHz, CDCl₃): δ 1.55 (s,3H), 1.59 (s,3H), 2.95 (s,3H), 5.99 (s, 1H), 6.53 (m, 1H), 6.62 (m, 1H), 6.80 (m, 1H), 7.19 (m, 1H), 7.54 (m, 1H), 7.71 (m, 1H), 7.81 (d, 1H, J=8.4 Hz), 8.12 (d,J=8.4 Hz, 1H), 8.18 (d,J=2.1 Hz, 1H), 8.93 (d,J=2.1 Hz, 1H); MS (APCI, m/z): 305 ([M+1]+)

### [Example 3]

### Production of 3-(4,4-dimethyl-1-isochromanyl)-quinoline

Ph₃P (56.9 mg, 0.21 mmol), HCO₂Na (55.3 mg, 0.82 mmol) and Et₄NCl (89.9 mg, 0.54 mmol) were added to a CH₃CN (10 mL) solution of 3-[(2-bromophenyl)-(2-methylallyloxy)-methyl]-quinoline (0.20 g, 0.54 mmol), and after replacing the inside of the reaction vessel with nitrogen, Pd(OAc)₂ (24.7 mg, 0.11 mmol) were added followed by heating and refluxing for 16 hours. After allowing to cool to room temperature, ethyl acetate (50 mL) and water (30 mL) were added to the reaction solution, and the aqueous layer was extracted with ethyl acetate (60 mL). The combined organic layer was washed with brine (50 mL) and dried with magnesium sulfate. After filtering, the solvent was distilled off under reduced pressure and the resulting residue was purified by silica gel column chromatography (silica gel 80 g, n-hexane:ethyl acetate = 4:1 to 1:1) to obtain 0.10 g (64%) of the target compound.
Physical properties: mp 103 to 105°C
¹H NMR (300 MHz, CDCl₃) : δ 1.35 (s,3H), 1.47 (s,3H), 3.71 (d,J=11.4 Hz, 1H) 3.78 (d,J=11.4 Hz, 1H), 5.98 (s, 1H), 6.71 (m, 1H), 7.06 (m, 1H), 7.27 (m, 1H), 7.44 (m, 1H), 7.54 (m, 1H), 7.72 (m, 1H), 7.79 (d,J=8.1 Hz, 1H), 8.02 (d, 1H, J=2.1 Hz), 8.12 (d, 1H, J=8.1 Hz), 8.91 (d,J=2.1 Hz, 1H); MS (APCI, m/z): 290 ([M+1]+)

### [Example 4]

### Production of 3-(8-fluoro-2,2-dimethyl-4H-benzo[1,3] dioxin-4-yl)-quinoline

Pyridinium 4-toluenesulfonate (18.6 mg, 0.074 mmol) was added to a 2,2-dimethoxypropane (5 mL) solution of 2-fluoro-6-(hydroxy-quinolin-3-yl-methyl)-phenol (0.20 g, 0.74mmol) followed by heating and refluxing for 18 hours. After allowing to cool to room temperature, saturated aqueous sodium bicarbonate solution (10 mL) was added to the reaction vessel followed by pouring the entire contents into ethyl acetate (60 mL) and water (60 mL). The aqueous layer was extracted with ethyl acetate (60 mL), and the combined organic layer was washed with brine (30 mL) and dried with magnesium sulfate. After filtering, the solvent was distilled off under reduced pressure and the resulting residue was purified by silica gel column chromatography (silica gel 80 g, n-hexane:ethyl acetate = 4:1) to obtain 0.15 g (66%) of the target compound.
Physical properties: mp 117 to 119°C
¹H NMR (300 MHz, CDCl₃): δ 1.73 (s,3H), 1.74 (s,3H), 6.09 (s, 1H), 6.43 (m, 1H), 6.71 (m, 1H), 7.00 (m, 1H), 7.57 (m, 1H), 7.74 (m, 1H), 7.82 (dd, J=1.3, 8.4 Hz, 1H), 8.12 (d,J=8.4 Hz, 1H) 8.16 (d,J=2.4 Hz, 1H), 8.88 (d,J=2.4 Hz, 1H); MS (APCI, m/z): 310

### ([M+1]+)

### [Example 5]

### Production of 3-(3,3-dimethyl-1-hydroxy-isochroman-1-yl) quinoline

A 1.7 M t-BuLi solution (2.69 mL, 5.04 mmol) was added to a diethyl ether solution (20 mL) of 3-bromoquinoline (1.00 g, 4.80 mmol) at -78°C followed by stirring for 1.5 hours at that temperature. A diethyl ether solution (2 mL) of 3,3-dimethyl-1-isochromanone (0.85 g, 4.80 mmol) was added to this solution followed by stirring for 1 hour at -78°C and 1 hour at -50°C. Subsequently, the reaction solution was poured into diethyl ether (50 mL), water (50 mL) and saturated aqueous ammonium chloride solution (100 mL) and the aqueous layer was extracted with ethyl acetate (100 mL). The combined organic layer was washed with brine (50 mL) and dried with magnesium sulfate. After filtering, the solvent was distilled off under reduced pressure and the resulting residue was purified by silica gel column chromatography (silica gel 160 g, n-hexane:ethyl acetate = 2:1) to obtain 0.76 g (52%) of the target compound.
Physical properties: mp 143 to 145°C
¹H NMR (300 MHz, CDCl₃) : δ 1.28 (s, 6H), 3. 08 (s, 2H), 7.32-7.46 (m,3H), 7.52-7.71 (m,2H), 7.83-7.91 (m,2H), 8.18 (dd,J=8.7 Hz, 1H), 8.51 (d, J=2.1 Hz, 1H), 9.31 (d, J=2.1 Hz, 1H); MS (APCI, m/z): 306 ([M+1]+)

### [Example 6]

### Production of 3-(3,3-dimethylisochroman-1-yl)-quinoline

Et₃SiH (0.23g, 1.96mmol) and CF₃CO₂H (0.15g, 1.3mol) were added to a CH₃CN solution (8 ml) of 3-(3,3-dimethyl-1-hydroxy-isochroman-1-yl)-quinoline (0.20 g, 0.65 mmol) followed by stirring for 20 hours at room temperature and additionally stirring for 14 hours at 80 to 90°C. Subsequently, the reaction solution was poured into ethyl acetate (100 mL), water (30 mL) and saturated aqueous sodium bicarbonate solution (50 mL), and the aqueous layer was extracted with ethyl acetate (30 mL). The combined organic layer was washed with brine (20 mL) and dried with magnesium sulfate. After filtering, the solvent was distilled off under reduced pressure and the resulting residue was purified by silica gel column chromatography (silica gel 80 g, n-hexane:ethyl acetate = 8:1 to 6:1) to obtain 0.12 g (64%) of the target compound.
Physical properties: amorphous
¹H NMR (300 MHz, CDCl₃): δ 1.37 (s,3H), 1.44 (s,3H), 2.72 (d,J=16.2 Hz, 1H), 3.14 (d,J=16.2 Hz, 1H), 5.93 (s, 1H), 6.72 (m, 1H), 7.05 (m, 1H), 7.15-7.21 (m,2H), 7.53 (m, 1H), 7.70 (m, 1H), 7.80 (dd,J=1.2, 8.1 Hz, 1H), 8.09-8.12 (m,2H), 8.85 (d,J=2.1 Hz, 1H); MS (APCI, m/z): 290 ([M+1]+)

### [Example 7]

### Production of 3-(3,3-dimethyl-1-methoxy-isochroman-1-yl) -quinoline

MeSO₃H (88.1 mg, 0.91 mmol) was added to a CH₃OH solution (8 mL) of 3-(3,3-dimethyl-1-hydroxy-isochroman-1-yl)-quinoline (0.28 g, 0.91 mmol) followed by stirring for 20 hours at room temperature. Subsequently, the reaction solution was poured into ethyl acetate (50 mL), water (50 mL) and saturated aqueous sodium bicarbonate solution (100 mL), and the aqueous layer was extracted with ethyl acetate (60 mL). The combined organic layer was washed with brine (50 mL) and dried with magnesium sulfate. After filtering, the solvent was distilled off under reduced pressure, and the resulting residue was purified by silica gel column chromatography (silica gel 80 g, n-hexane:ethyl acetate = 4:1) to obtain 0.24 g of the target compound (83%).
Physical properties: amorphous
¹H NMR (300 MHz, CDCl₃): δ 1.43 (s,3H), 1.52 (s,3H), 2.89 (d, J=15.6 Hz, 1H), 3.04 (d, J=15.6 Hz, 1H), 3.40 (s,3H), 7.08-7.21 (m,3H), 7.30 (m, 1H), 7.53 (m, 1H), 7.69 (m, 1H), 7.84 (d,J=8.1 Hz, 1H), 8.08 (d,J=8.1 Hz, 1H) 8.27 (d,J=2.1 Hz, 1H), 9.13 (d,J=2.1 Hz, 1H); MS (APCI, m/z): 320 ([M+1]+)

Other compounds of the present invention were produced in the same manner as the above examples. The compound numbers (corresponding to the compound numbers of Tables 1 to 8), compound names, physical properties and NMR data are indicated below.

### [Compound No. 1-3]

### 3-(2,2-dimethyl-4H-benzo[1,3]dioxin-4-yl)-quinoline

Physical properties: 130 to 132°C
¹H NMR (300 MHz, CDCl₃): δ 1.69 (s,6H), 6.09 (s, 1H), 6.65 (m, 1H), 6.78 (m, 1H), 6.91 (dd,J=8.4,1.2 Hz, 1H), 7.19 (m, 1H), 7.55 (m, 1H), 7.72 (m, 1H), 7.84 (dd,J=8.1,1.2 Hz, 1H), 8.12 (d,J=8.4 Hz, 1H), 8.16 (d,J=2.1 Hz, 1H) 8.89 (d,J=2.1 Hz, 1H); MS (APCI, m/z): 291 ([M+1]+)

### [Compound No. 1-35]

### 2-chloro-5-(2,2-dimethyl-4H-benzo[1,3]dioxin-4-yl)-3-methylpyridine

Physical properties: refractive index n_{D} (22.2): 1.5692
¹H NMR (300 MHz, CDCl₃): δ 1.63 (s,3H), 1.65 (s,3H), 2.34 (s,3H), 5.87 (s,1H), 6.60 (d,1H,J=7.8 Hz), 6.82-6.89 (m,2H), 7.19 (t,1H,J=7.4 Hz), 7.49 (d,1H,J=2.1 Hz), 8.30 (d,1H,J=2.1 Hz)

### [Compound No. 1-45]

### 3-(2,2-dimethyl-4H-benzo[1,3]dioxin-4-yl)-5,6,7,8-tetrahydroquinoline

Physical properties: 149 to 150°C
¹H NMR (300MHz, CDCl₃) : δ1.39 (s,3H), 1.42 (s,3H), 1.75-1.86 (m,4H), 2.72 (t,1H,J=6.3Hz), 2.88 (t,1H,J=6.3Hz), 5.94 (s,1H), 6.82-6.88 (m,2H), 7.02 (d,1H,J=7.8 Hz), 7.15 (t,1H,J=7.6 Hz), 7.26-7.34 (m,1H), 8.32 (s,1H), 8.41 (s,1H)

### [Compound No. 1-50]

### 5-(2,2-dimethyl-4H-benzo[1,3]dioxin-4-yl)-thieno[2,3-b] pyridine

Physical properties: 133 to 134°C
¹H NMR (300 MHz, CDCl₃) : δ1.67 (s,6H), 6.05 (s,1H), 6.63 (d,1H,J=7.5 Hz), 6.80 (d,1H,J=7.5 Hz), 6.89 (d,1H,J=8.4 Hz), 7.16-7.19 (m,1H), 7.22 (d,1H,J=3.3 Hz), 7.54 (d,1H,J=3.3 Hz), 8.05 (d,1H,J=2.1 Hz), 8.62 (d,1H,J=2.1 Hz)

### [Compound No. 2-7]

### 3-(2-ethyl-8-fluoro-2-methyl-4H-benzo[1,3]dioxin-4-yl)-quinoline

Physical properties: amorphous
¹H NMR (300 MHz, CDCl₃) : δ1.12 (t,J=7.4 Hz,3H), 1.67 (s,3H), 2.02 (q,J=7.4Hz,2H), 6.10 (s,1H), 6.43 (d,J=7.7 Hz), 6.71 (m,1H), 7.00 (m,1H), 7.58 (m,1H), 7.75 (m,1H), 7.84 (d,J=8.0 Hz,1H), 8.12-8.18 (m,2H), 8.90 (s,1H); MS (APCI, m/z): 324 ([M+1]+)

### [Compound No. 2-17]

### 8-fluoro-3-(8-fluoro-2,2-dimethyl-4H-benzo[1,3]dioxin-4-yl)-quinoline

Physical properties: 154 to 156°C
¹H NMR (300 MHz, CDCl₃): δ 1.73 (s,3H), 1.75 (s,3H), 6.11 (s,1H) 6.42 (d,J=7.8 Hz,1H), 6.72 (m,1H), 7.42 (m,1H), 7.50 (m,1H), 7.62 (d,J=8.1 Hz,1H), 8.19 (m,1H), 8.94 (d,J=2.1 Hz,1H); MS (APCI, m/z): 328 ([M+1]+)

### [Compound No. 2-32]

### 3-(8-chloro-2,2-dimethyl-4H-benzo[1,3]dioxin-4-ylquinoline

Physical properties: amorphous
¹H NMR (300 MHz, CDCl₃) : δ 1.71 (s,3H), 1.76 (s,3H), 6.08 (s,1H), 6.56 (d,J=7.8Hz), 6.72 (m,1H), 7.28 (m,1H), 7.57 (m,1H), 7.74 (m,1H), 7.82 (d,J=7.8 Hz,1H), 8.11-8.16 (m,2H), 8.88 (d,J=2.1 Hz,1H)

### [Compound No. 2-33]

### 8-fluoro-3-(8-chloro-2,2-dimethyl-4H-benzo[1,3]dioxin-4-yl)-quinoline

Physical properties: 170 to 171°C
¹H NMR (300 MHz, CDCl₃): δ 1.71 (s,3H), 1.76 (s,3H), 6.10 (s,1H), 6.53-6.57 (m,1H), 6.73 (t,1H,J=8.0Hz), 7.25-7.63 (m,4H), 8.19 (t,1H,J=1.8 Hz), 8.94 (d,1H,J=1.8 Hz)

### [Compound No. 6-14]

### 3-(3,3-dimethylisochroman-1-yl)-8-fluoro-quinoline Physical properties: amorphous

¹H NMR (300 MHz, CDCl₃): δ 1.37 (s,3H), 1.44 (s,3H), 2.72 (d,J=15.9 Hz,1H), 3.13 (d,J=15.9 Hz,2H), 5.94 (s,1H), 6.71 (d,J=7.8 Hz,1H), 7.06 (m,1H), 7.15-7.22 (m,2H), 7.38 (m,1H), 7.47 (m,1H), 7.59 (d,J=8.1 Hz,1H), 8.14 (m,1H), 8.91 (d,J=1.8 Hz,1H); MS (APCI, m/z): 308 ([M+1]+)

### [Compound No. 6-22]

### 3-(5-fluoro-3,3-dimethylisochroman-1-yl)-quinoline Physical properties: amorphous

¹H NMR (300 MHz, CDCl₃): δ 1.38 (s,3H), 1.48 (s,3H), 2.90 (s,2H), 5.92 (s,1H), 6.52 (d,J=7.4 Hz,1H), 6.90-7.06 (m,2H), 7.55 (m,1H), 7.71 (m,1H), 7.81 (dd,J=1.2,8.3 Hz,1H), 8.09-8.12 (m,2H), 8.83 (d,J=2.1 Hz,1H); MS (APCI, m/z): 308 ([M+1]+)

### [Compound No. 6-28]

### 8-fluoro-3-(5-fluoro-3,3-dimethylisochroman-1-yl)-quinoline

Physical properties: amorphous
¹H NMR (300 MHz, CDCl₃): δ 1.38 (s,3H), 1.48 (s,3H), 2.89 (s,2H), 5.93 (s,1H), 6.51 (d,J=7.8 Hz,1H), 6.93 (m,1H), 7.01 (m,1H), 7.39 (m,1H), 7.48 (m,1H), 7.60 (d,J=7.5 Hz,1H), 8.14 (m,1H), 8.89 (d,J=1.8 Hz,1H); MS (APCI, m/z): 326 ([M+1]+)

Although the following indicates some preparation examples of the fungicide of the present invention, the additives and addition ratios are not limited to these examples and can be varied over a wide range. The term "parts" indicated in the preparation examples refers to parts by weight.

### Preparation Example 1 Wettable Powder

| | |
|---|---|
| Compound of present invention | 40 parts |
| Clay | 48 parts |
| Sodium dioctylsulfosuccinate | 4 parts |
| Sodium lignin sulfonate | 8 parts |

The above components are uniformly mixed and finely crushed to obtain a wettable powder containing 40% of the active ingredient.

| | |
|---|---|
| Compound of present invention | 10 parts |
| Solvesso 200 | 53 parts |
| Cyclohexanone | 26 parts |
| Calcium dodecylbenzenesulfonate | 1 part |
| Polyoxyethylene alkyl allyl ether | 10 parts |

The above components are mixed and dissolved to obtain an emulsion containing 10% of the active ingredient.

### Preparation Example 3 Powder

| | |
|---|---|
| Compound of present invention | 10 parts |
| Clay | 90 parts |

The above components are uniformly mixed and finely crushed to obtain a powder containing 10% of the active ingredient.

### Preparation Example 4 Granules

| | |
|---|---|
| Compound of present invention | 5 parts |
| Clay | 73 parts |
| Bentonite | 20 parts |
| Sodium dioctylsulfosuccinate | 1 part |
| Potassium phosphate | 1 part |

The above components are crushed and mixed well followed by the addition of water, mixing well, granulating and drying to obtain granules containing 5% of the active ingredient.

### Preparation Example 5 Suspension

| | |
|---|---|
| Compound of present invention | 10 parts |
| Polyoxyethylene alkyl allyl ether | 4 parts |
| Sodium polycarbonate | 2 parts |
| Glycerin | 10 parts |
| Xanthan gum | 0.2 parts |
| Water | 73.8 parts |

The above components are mixed followed by wet-crushing to a particle diameter of 3 microns or less to obtain a suspension containing 10% of the active ingredient.

### Preparation Example 6 Water Dispersible Granules

| | |
|---|---|
| Compound of present invention | 40 parts |
| Clay | 36 parts |
| Potassium chloride | 10 parts |
| Sodium alkylbenzenesulfonate | 1 part |
| Sodium lignin sulfonate | 8 parts |
| Formaldehyde condensation product of sodium alkylbenzenesulfonate | 5 parts |

The above components are uniformly mixed and finely crushed followed by adding a suitable amount of water and mixing to form a clay-like mixture. The clay-like mixture is granulated and dried to obtain water dispersible granules containing 40% of the active ingredient.

### (Test Example 1) Apple Scab Control Test

Emulsions of fungicides of the present invention were sprayed at an active ingredient concentration of 100 ppm onto apple seedlings (variety: Ralls Janet, leaf stage: 3 to 4) cultivated in unglazed pots. After allowing to air-dry at room temperature, the seedlings were inoculated with conidiospores of apple scab pathogen (Venturia inaequalis) followed by holding for 2 weeks indoors at 20°C and high humidity using a 12 hour light/dark cycle. The appearance of lesions on the leaves was compared with untreated seedlings to determine control effects.
As a result, the following compounds demonstrated superior control values of 75% or more:
compound numbers (corresponding to the compound numbers listed in Tables 1 to 8): 1-3, 1-50, 2-3, 2-7, 2-17, 2-32, 2-33, 6-3, 6-14, 6-22, 6-28, 7-1, 8-20.

### (Test Example 2) Cucumber Gray Mold Control Test

Emulsions of the compound of the present invention were sprayed at an active ingredient concentration of 100 ppm onto cucumber seedlings (variety: Sagami Hanjiro, leaf stage: cotyledon) cultivated in unglazed pots. After allowing to air-dry at room temperature, the seedlings were drip-inoculated with conidiospore suspensions of cucumber gray mold pathogen (Botrytis cinerea) followed by holding in the dark for 4 days indoors at 20°C and high humidity. The appearance of lesions on the leaves was compared with untreated seedlings to determine control effects.

As a result, the following compounds demonstrated superior control values of 75% or more:
compound numbers (corresponding to the compound numbers listed in Tables 1 to 8): 1-3, 1-50, 2-3, 2-7, 2-17, 2-32, 2-33, 6-3, 6-14, 6-22, 6-28, 7-1, 8-3, 8-20.

## Claims

1. A nitrogen-containing heterocyclic compound represented by the following formula (I), or a salt thereof: 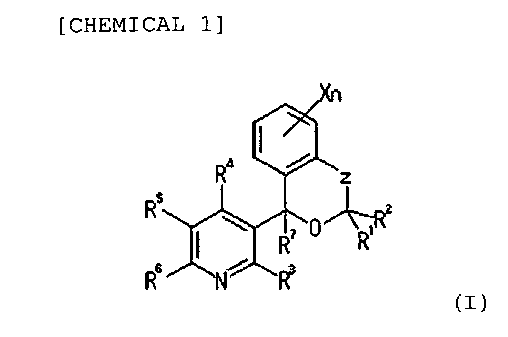 wherein,
R¹ and R² each independently represent a hydrogen atom, halogen atom, C1-20 alkyl group, C1-20 haloalkyl group, C2-20 alkenyl group, C2-20 haloalkenyl group, C2-20 alkynyl group, C2-20 haloalkynyl group, C1-20 alkoxy group, C1-20 haloalkoxy group, C3-20 cycloalkyl group, C4-20 cycloalkenyl group, C8-20 cycloalkynyl group, C1-20 acyl group or C1-20 alkoxycarbonyl group, and R¹ and R² may bond together to form an unsubstituted or substituted 5- to 8-membered ring;
R³ to R⁶ each independently represent a hydrogen atom, halogen atom, C1-20 alkyl group, C1-20 haloalkyl group, C2-20 alkenyl group, C2-20 haloalkenyl group, C2-20 alkynyl group, C2-20 haloalkynyl group, C1-20 alkoxy group, C1-20 haloalkoxy group, C3-20 cycloalkyl group, C3-20 cycloalkoxy group, C2-20 alkenyloxy group, C2-20 alkynyloxy group, C1-20 alkylthio group, cyano group, nitro group, unsubstituted or substituted amino group, unsubstituted or substituted aryl group, unsubstituted or substituted aralkyl group, unsubstituted or substituted aryloxy group or unsubstituted or substituted heterocyclic group, and R⁴ and R⁵, and R⁵ and R⁶ may bond together to form an unsubstituted or substituted 5- to 8-membered ring;
R⁷ represents a hydrogen atom, halogen atom, hydroxyl group, C1-20 alkyl group, C1-20 haloalkyl group, C2-20 alkenyl group, C2-20 haloalkenyl group, C2-20 alkynyl group, C2-20 haloalkynyl group, C1-20 alkoxy group, C1-20 haloalkoxy group, C3-20 cycloalkyl group, C3-20 cycloalkoxy group, C2-20 alkenyloxy group, C2-20 alkynyloxy group, C1-20 alkylthio group or cyano group;
X represents a halogen atom, nitro group, unsubstituted or substituted amino group, hydroxyl group or organic group;
n represents an integer of 0 to 4;
Z represents an oxygen atom, sulfur atom, group represented by the formula: CR⁸R⁹ or the formula: NR¹⁰;
R⁸ and R⁹ each independently represent a hydrogen atom, halogen atom, C1-20 alkyl group, C1-20 haloalkyl group, C2-20 alkenyl group, C2-20 haloalkenyl group, C2-20 alkynyl group, C2-20 haloalkynyl group, C1-20 alkoxy group, C1-20 haloalkoxy group, C3-20 cycloalkyl group, C4-20 cycloalkenyl group, C8-20 cycloalkynyl group, C1-20 acyl group or C1-20 alkoxycarbonyl group, and R⁸ and R⁹ may bond together to form an unsubstituted or substituted 5- to 8-membered ring; and,
R¹⁰ represents a hydrogen atom, C1-20 alkyl group, C2-20 alkenyl group, C2-20 alkynyl group, C1-20 acyl group, C1-20 alkoxycarbonyl group, C1-20 alkylsulfonyl group, C1-20 alkoxy C1-20 alkyl group, C2-20 acyloxy C1-20 alkyl group, unsubstituted or substituted aminocarbonyl group, unsubstituted or substituted arylsulfonyl group or unsubstituted or substituted aminosulfonyl group.

2. The nitrogen-containing heterocyclic compound or salt thereof according to claim 1, wherein R⁵ and R⁶ bond together to form an aromatic ring.

3. A fungicide for agricultural and horticultural use that contains at least one of the nitrogen-containing heterocyclic compound or salt thereof according to claim 1 or 2 as an active ingredient thereof.
